# EUROPÄISCHE PATENTSCHRIFT

(11) **EP 0 666 848 B1**
(45) Veröffentlichungstag und Bekanntmachung des Hinweises auf die Patenterteilung: **30.12.1998**
(21) Anmeldenummer: 94900070.7
(22) Anmeldetag: 19.10.1993
(51) Int. Cl.: C07D 211/88, C07D 211/86, C07C 233/34, C07C 233/54, A01N 43/40

(54) **SUBSTITUIERTE N-PHENYLGLUTARIMIDE UND N-PHENYLGLUTARSÄUREAMIDE, DEREN HERSTELLUNG UND VERWENDUNG IN DER LANDWIRTSCHAFT**
SUBSTITUTED N-PHENYLGLUTARIMIDES AND N-PHENYLGLUTARIC ACID AMIDES, THEIR PREPARATION AND USE IN AGRICULTURE
N-PHENYLGLUTARIMIDES SUBSTITUES ET AMIDES D'ACIDE N-PHENYLGLUTARIQUE, LEUR PREPARATION ET LEUR UTILISATION DANS L'AGRICULTURE

(30) Priorität: 31.10.1992 DE 4236880
(43) Veröffentlichungstag der Anmeldung: 16.08.1995
(73) Patentinhaber: BASF Aktiengesellschaft, 67063 Ludwigshafen (DE)
(72) Erfinder: KLINTZ, Ralf, D-67125 Dannstadt-Schauernheim (DE); HAMPRECHT, Gerhard, D-69469 Weinheim (DE); HEISTRACHER, Elisabeth, D-67061 Ludwigshafen (DE); SCHAEFER, Peter, D-67098 Bad Duerkheim (DE); KARDORFF, Uwe, D-68159 Mannheim (DE); WESTPHALEN, Karl-Otto, D-67346 Speyer (DE); GERBER, Matthias, D-67117 Limburgerhof (DE); WALTER, Helmut, D-67283 Obrigheim (DE)
(86) Internationale Anmeldenummer: EP9302879
(87) Internationale Veröffentlichungsnummer: WO9410147

(56) Entgegenhaltungen:
- EP-A- 0 415 642
- EP-A- 0 454 444
- EP-A- 0 468 924
- US-A- 3 655 669

## Beschreibung

Die vorliegende Erfindung betrifft neue substituierte N-Phenylglutarimide der Formel I 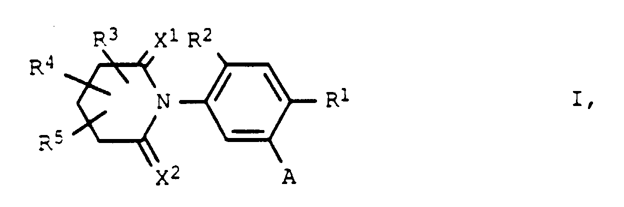 in der die Variablen folgende Bedeutung besitzen:
X¹, X²
   Sauerstoff oder Schwefel;
R¹
   Halogen, Nitro, Cyano oder Trifluormethyl;
R²
   Wasserstoff oder Halogen;
R³, R⁴, R⁵
   - unabhängig voneinander Wasserstoff, Halogen, Cyano, C₁-C₆-Alkyl, C₃-C₇-Cycloalkyl, C₂-C₆-Alkenyl, C₂-C₆-Alkinyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Cyanoalkyl, C₁-C₆-Alkoxycarbonyl, Phenyl oder Benzyl, wobei die Phenylgruppe bzw. der Phenylring der Benzylgruppe ggf. substituiert ist durch C₁-C₆-Alkyl, C₁-C₆-Alkoxy, Halogen, Cyano, Nitro, Trifluormethyl,
   - oder zwei Substituenten eines Kohlenstoffatoms des Glutarimidrings sind über eine 2- bis 5-gliedrige Kette miteinander verbunden und formen so einen Spiroring, der gewünschtenfalls ein oder zwei Halogenatome tragen kann, wobei der Spiroring neben C-Atomen auch ein oder zwei nicht benachbarte Ringglieder enthalten kann, die ausgewählt sind aus der Gruppe bestehend aus -O-, -S-, -NH- und -N(C₁-C₄-Alkyl)-,
   - oder zwei Substituenten zweier benachbarter Kohlenstoffatome des Glutarimidrings sind über eine 1- bis 5-gliedrige Kette miteinander verbunden und bilden so einen ankondensierten Ring, der gewünschtenfalls ein oder zwei Halogenatome tragen kann, wobei der ankondensierte Ring neben C-Atomen auch ein oder zwei nicht benachbarte Ringglieder enthalten kann, die ausgewählt sind aus der Gruppe bestehend -O-, -S-, -NH- und -N(C₁-C₄-Alkyl)-;

A
   CHR⁶-CHR⁷-CO-B oder CR⁶=CR⁸-CO-B, wobei
   - R⁶ Wasserstoff, C₁-C₆-Alkyl oder C₁-C₆-Halogenalkyl;
   - R⁷ Halogen, C₁-C₆-Halogenalkyl, Hydroxy, C₁-C₆-Alkoxy oder C₁-C₆-Alkylcarbonyloxy, und
   - R⁸ Wasserstoff, Halogen, Cyano, C₁-C₆-Alkyl, C₁-C₆-Halogenalkyl, Hydroxy, C₁-C₆-Alkoxy, C₁-C₆-Alkylcarbonyl, C₁-C₆-Alkoxycarbonyl oder C₁-C₆-Alkylcarbonyloxy
   bedeuten und wobei
B für eine der folgenden Bedeutungen steht:
   (a) Wasserstoff,
   (b) C₁-C₆-Alkyl, C₃-C₆-Alkenyl, C₃-C₆-Alkinyl, C₁-C₆-Halogenalkyl, C₃-C₇-Cycloalkyl, C₁-C₆-Alkoxy-C₁-C₆-alkyl, C₁-C₆-Dialkoxy-C₁-C₆-alkyl oder C₁-C₆-Alkylthio-C₁-C₆-alkyl;
   (c) OR⁹, SR⁹, wobei R⁹
      Wasserstoff, C₁-C₆-Alkyl, C₃-C₆-Alkenyl, C₃-C₆-Alkinyl, C₃-C₇-Cycloalkyl, Halogen-C₁-C₆-Alkyl, C₁-C₆-Cyanoalkyl, C₃-C₆-Halogenalkenyl, C₁-C₆-Alkoxycarbonyl-C₁-C₆-alkyl, C₁-C₆-Alkoxy-C₁-C₆-alkyl, C₁-C₆-Alkylthio-C₁-C₆-alkyl, C₁-C₆-Alkylimino oder C₁-C₆-Alkyloximino-C₁-C₆-alkyl, Phenyl, Phenyl substituiert durch einen oder mehrere Reste C₁-C₆-Alkyl, C₃-C₆-Alkenyl, Halogen, Cyano, Nitro, C₁-C₆-Alkoxy oder C₁-C₆-Alkoxycarbonyl, Benzyl, Benzyl substituiert durch einen oder mehrere Reste C₁-C₆-Alkyl, C₃-C₆-Alkenyl, Halogen, Cyano, Nitro, C₁-C₆-Alkoxy oder C₁-C₆-Alkoxycarbonyl bedeutet;
   (d) NR¹⁰R¹¹, wobei R¹⁰ und R¹¹ unabhängig voneinander folgende Bedeutung haben:
      Wasserstoff, C₁-C₆-Alkyl, C₃-C₆-Alkenyl, C₃-C₆-Alkinyl, C₃-C₆-Cycloalkyl, C₁-C₆-Halogenalkyl, C₁-C₆-Alkylcarbonyl, C₁-C₆Alkoxycarbonyl, C₁-C₆-Alkoxy-C₁-C₆-alkyl, Phenyl, Phenyl substituiert durch einen bis drei Reste C₁-C₆-Alkyl, C₃-C₆-Alkenyl, Halogen, Cyano, Nitro, C₁-C₆-Alkoxy oder C₁-C₆-Alkoxycarbonyl, oder R¹⁰ und R¹¹ zusammen mit dem Stickstoffatom, an das sie gebunden sind, einen gesättigten oder ungesättigten 4- bis 7-gliedrigen Heterocyclus mit einem oder zwei weiteren gleichen oder verschiedenen Heteroatomen ausgewählt aus der Gruppe Stickstoff, Sauerstoff oder Schwefel;
   sowie die landwirtschaftlich brauchbaren Salze der substituierten N-Phenylglutarimide I.

Insbesondere betrifft die Erfindung substituierte N-Phenylglutarimide der Formel I, in der R³ C₁-C₆-Alkyl oder -Halogenalkyl und R⁴, R⁵ Wasserstoff oder C₁-C₄-Alkyl bedeuten sowie Glutarimide der Formel I' 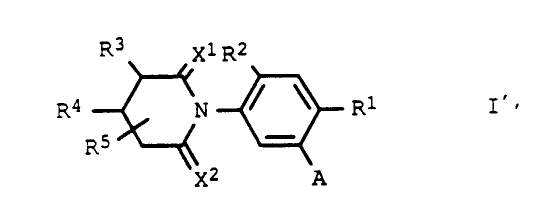 wobei R⁴ C₁-C₆-Halogenalkyl, R³ und R⁵ Wasserstoff, C₁-C₆-Alkyl oder C₁-C₆-Halogenalkyl bedeuten und A, X¹, X² wie voranstehend definiert sind.

Außerdem betrifft die Erfindung herbizide und desiccant und/oder defoliant wirksame Mittel, welche diese Verbindungen als wirksame Substanzen enthalten.

Ein weiterer Aspekt der Erfindung betrifft N-Phenylglutarsäureamide der allgemeinen Formel II 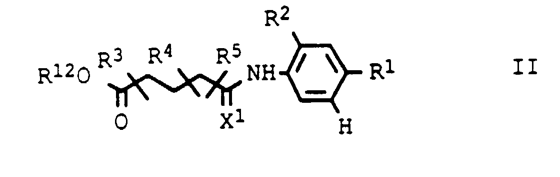 in der die Variablen X¹, R¹, R², R³, R⁴, R⁵ und A die gleiche Bedeutung besitzen, wie oben angeführt und R¹² Wasserstoff, C₁-C₆-Alkyl oder Benzyl bedeutet.

Die N-Phenylglutarsäureamide II dienen als Zwischenprodukte zur Herstellung der substituierten N-Phenylglutarimide I, lassen sich aber auch als Herbizide, Bioregulatoren und Mittel zur Abszission und/oder Defoliation von Pflanzenorganen verwenden.

Aus den Offenlegungsschriften EP-A 391 847, EP-A 415 641, EP-A 415 642 und EP-A 454 444 sind sowohl herbizid wirksame, speziell substituierte N-Phenylglutarimide als auch speziell substituierte N-Phenylglutarsäureamide bekannt. In der Offenlegungsschrift WO 87/ 07 602 werden N-Phenyl-substituierte Glutarimide allgemein erwähnt .

Der Wirkungsgrad und die Selektivität dieser bekannten Herbizide bezüglich der Schadpflanzen vermag jedoch nur bedingt zu befriedigen, so daß der Erfindung neue herbizid wirksame Verbindungen als Aufgabe zugrunde lagen, mit denen sich - bei guter Verträglichkeit für die Nutzpflanzen - Schadpflanzen besser als bisher gezielt bekämpfen lassen.

Demgemäß wurden die eingangs definierten substituierten N-Phenyllutarimide I und die N-Phenylglutarsäureamide II gefunden.

Ferner wurden Mittel gefunden, die diese Substanzen enthalten und eine gute herbizide Wirkung besitzen.

Außerdem wurde gefunden, daß sich die erfindungsgemäßen Verbindungen I und II als Defoliations- und Desikkationsmittel eignen, z.B. in Baumwolle, Kartoffel, Raps, Sonnenblumen, Sojabohnen oder Ackerbohnen.

Die für die Substituenten R¹ bis R⁵ genannten Bedeutungen stellen Sammelbegriffe für individuelle Aufzählungen der einzelnen Gruppenmitglieder dar. Sämtliche Alkyl-, Alkenyl-, Alkinyl- und Halogenalkyl-Teile können geradkettig oder verzweigt sein. Die Halogenalkyl- und Halogenalkoxyreste können gleiche oder verschiedene Halogenatome tragen.

Im einzelnen bedeuten beispielsweise:
x¹, x²
   Sauerstoff oder Schwefel, vorzugsweise X¹ und X² gleichzeitig Sauerstoff oder X¹ Schwefel und X² Sauerstoff;
R¹
   Nitro, Cyano, Trifluormethyl oder Halogen wie Fluor, Chlor, Brom oder Iod, vorzugsweise Halogen, insbesondere Chlor;
R²
   Wasserstoff oder Halogen wie für R¹ angegeben, vorzugsweise Wasserstoff, Chlor und Fluor;
R³, R⁴, R⁵
   unabhängig voneinander
   Wasserstoff,
   Halogen wie für R¹ genannt, Cyano,

C₁-C₆-Alkyl, insbesondere C₁-C₄-Alkyl: Methyl, Ethyl, n-Propyl, Isopropyl, n-Butyl, sek.-Butyl, tert.-Butyl,

C₃-C₇-Cycloalkyl: Cyclopropyl, Cyclobutyl, Cyclopentyl, Cyclohexyl und Cycloheptyl,

C₂-C₆-Alkenyl oder C₂-C₆-Alkinyl, insbesondere C₂-C₄-Alkenyl oder -Alkinyl: z.B. Vinyl, Allyl, Ethinyl, Propargyl,

C₁-C₆-Halogenalkyl: Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl und 3-Chlorpropyl, vorzugsweise Trifluormethyl,

C₁-C₆-Alkoxy und -Halogenalkoxy, insbesondere C₁-C₄-Alkoxy und C₁-C₄-Halogenalkoxy: z.B. Methoxy, Ethoxy, n-Propoxy, 1-Methylethoxy, n-Butoxy, 1-Methylpropoxy, 2-Methylpropoxy, 1,1-Dimethylethoxy, n-Pentoxy, 1-Methylbutoxy, 2-Methylbutoxy, 3-Methylbutoxy, Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise C₁-C₂-Alkoxy oder C₁-C₂-Halogenalkoxy wie 2,2,2-Trifluorethoxy oder Methoxy,

C₁-C₆-, insbesondere C₁-C₄-Alkylthio und C₁-C₄-Halogenalkylthio: z.B. Methylthio, Ethylthio, n-Propylthio, 1-Methylethylthio, n-Butylthio, 1-Methyl-propylthio, 2-Methylpropylthio, 1,1-Dimethylethylthio, Chlormethylthio, Dichlormethylthio, Trifluormethylthio, Fluormethylthio, Difluormethylthio, Trifluormethylthio, 2,2,2-Trifluorethylthio und Pentafluorethylthio,

C₁-C₆-, insbesondere C₁-C₄-Cyanoalkyl: z.B. Cyanomethyl, 1-Cyanoeth-1-yl und 2-Cyanoeth-1-yl,

C₁-C₆-Alkoxycarbonyl, insbesondere C₁-C4-Alkoxycarbonyl, wobei die vorstehend für C₁-C₄-Alkoxy genannten Reste auch in der Kombination Alkoxycarbonyl bevorzugt sind: insbesondere bevorzugt Methoxy- und Ethoxycarbonyl,

Phenyl oder Benzyl, wobei der aromatische Kern jeweils substituiert sein kann, durch ein oder mehrere, insbesondere 1 bis 3 Reste, ausgewählt aus der Gruppe C₁-C₆-Alkyl, C₁-C₆-Alkoxy, insbesondere Methyl, Ethyl, Ethoxy oder Methoxy, Halogen wie Fluor, Chlor, Brom oder Iod, Cyano, Nitro und Trifluormethyl; beispielsweise Phenyl, 4-Fluorphenyl, 4-Chlorphenyl, 3-Fluorphenyl, 3-Chlorphenyl, 2-Fluorphenyl, 2-Chlorphenyl, 2,4-Dichlorphenyl, 4-Trifluormethylphenyl, 4-Methoxyphenyl, Benzyl oder 2,4-Dichlorbenzyl.

Zwei geminal gebundene Reste (R³+R⁴ oder R⁴+R⁵) können auch miteinander eine 2- bis 5-gliedrige Kette bilden (z.B. eine Ethylen-, Propylen-, Butylen- oder Pentylenkette) und so miteinander einen spiroanellierten Ring bilden, der unsubstituiert sein oder seinerseits 1 bis 2 Halogenatome wie Fluor, Chlor oder Brom tragen kann;

zwei vicinal gebundene Reste (R³+R⁴ oder R⁴+R⁵) können auch miteinander eine 1- bis 5-gliedrige Kette bilden (z.B. eine Methylen-, Ethylen-, Propylen-, Butylen- oder Pentylenkette) und so zusammen mit den C-Atomen, an die sie gebunden sind einen ankondensierten 3- bis 7-gliedrigen carbocyclischen Ring bilden, der unsubstituiert sein oder seinerseits 1 bis 2 Halogenatome wie Fluor, Chlor, Brom oder Iod tragen kann;
sowohl der Spiroring als auch der ankondensierte Ring können neben C-Atomen als Ringglieder noch ein oder zwei nicht benachbarte Ringglieder enthalten, und zwar jeweils -O-, -S-, -NH- oder -N(C₁-C₄-Alkyl)-.
A
   steht für eine Gruppierung 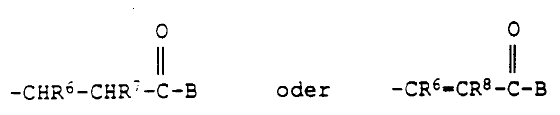 wobei R⁶ jeweils Wasserstoff, C₁-C₆-Alkyl, insbesondere C₁-C₄-Alkyl wie Methyl, Ethyl, n-Propyl, Isopropyl, n-Butyl, sek.-Butyl, tert.-Butyl, insbesondere Methyl und Ethyl, C₁-C₆-, insbesondere C₁- und C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor- 2,2-difluorethyl, 2,2-Dichlor-2-fluorethyl, 2,2,2-Trichlorethyl, Pentafluorethyl und 3-Chlorpropyl, vorzugsweise Trifluormethyl bedeutet und R⁷ für Halogen wie Fluor, Chlor, Brom oder Iod, insbesondere Chlor und Brom, für C₁-C₆-Halogenalkyl wie voranstehend für R⁶ genannt, insbesondere Trifluormethyl und Pentafluormethyl, für Hydroxy, für C₁-C₆-Alkoxy oder C₁-C₆-Alkylcarbonyloxy, wobei in beiden Gruppen die Alkylreste vorzugsweise 1 bis 4 C-Atome aufweisen, wie Methyl, Ethyl, n-Propyl, iso-Propyl, n-Butyl, iso-Butyl, sec.-Butyl, tert.-Butyl, also beispielsweise Methoxy, Ethoxy, Methoxycarbonyl und Ethoxycarbonyl steht.

R⁸ bedeutet vorzugsweise Wasserstoff, Cyano, Hydroxy, Halogen wie Fluor, Chlor, Brom oder Iod, insbesondere Chlor oder Brom, C₁-C₆-, insbesondere C₁-C₄-Alkyl wie Methyl, Ethyl, n-Propyl, iso-Propyl, n-Butyl, iso-Butyl, sec.-Butyl, tert.-Butyl, C₁-C₆-Halogenalkyl, insbesondere C₁-C₄-Halogenalkyl, wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor- 2,2-difluorethyl, 2,2-Dichlor-2-fluorethyl, 2,2,2-Trichlorethyl, Pentafluorethyl und 3-Chlorpropyl, besonders bevorzugt Trifluormethyl oder Alkoxy, Alkylcarbonyl, Alkoxycarbonyl oder Alkylcarbonyloxy, wobei die Alkyleinheiten in den vier letztgenannten Gruppen 1 bis 6, vorzugsweise 1 bis 4 C-Atome wie Methyl, Ethyl, n-Propyl, iso-Propyl, n-Butyl, iso-Butyl, sec.-Butyl, tert.-Butyl, aufweisen, also beispielsweise Methoxy, Ethoxy, Methylcarbonyl, Ethylcarbonyl, Methoxycarbonyl, Ethoxycarbonyl, Isopropoxycarbonyl, Methylcarbonyloxy oder Ethylcarbonyloxy.

Der Rest B ist sehr breit variierbar und bedeutet
(a) Wasserstoff;
(b) C₁-C₆-, insbesondere C₁-C₄-Alkyl wie Methyl, Ethyl, n-Propyl, iso-Propyl, n-Butyl, iso-Butyl, sek.-Butyl, tert.-Butyl; C₃-C₆-Alkenyl oder C₃-C₆-Alkinyl wie Allyl oder Propargyl; C₁-C₆-, insbesondere C₁-C₂-Halogenalkyl wie Trifluormethyl, 2,2,2-Trifluorethyl oder 2,2,2-Trichlorethyl; C₃-C₇-Cycloalkyl, insbesondere C₃-C₆-Cycloalkyl wie Cyclopropyl, Cyclopentyl oder Cyclohexyl; C₁-C₆-Alkoxy- oder -Dialkoxy-C₁-C₆-alkyl, insbesondere C₁-C₄-Alkoxy- oder -Dialkoxy-C₁-C₄-alkyl wie Methoxymethyl, Ethoxymethyl, Dimethoxymethyl, Diethoxymethyl oder i-Propoxymethyl; C₁-C₆-Alkylthio-C₁-C₆-alkyl, insbesondere C₁-C₄-Alkylthio-C₁-C₆-alkyl, z.B. Methylthiomethyl oder Ethylthiomethyl,
(c) OR⁹, SR⁹, wobei R⁹ folgende Bedeutung besitzt:
   Wasserstoff, C₁-C₆-, insbesondere C₁-C₄-Alkyl wie voranstehend für (b) genannt, C₃-C₆-Alkenyl und -Alkinyl, z.B. Allyl oder Propargyl; C₃-C₇-Cycloalkyl, z.B. Cyclopropyl, Cyclopentyl oder Cyclohexyl, C₁-C₆-Halogenalkyl wie 2,2,2-Trifluormethyl oder 2,2,2-Trichlorethyl; C₁-C₆-, insbesondere C₁-C₄-Cyanoalkyl, z.B. Cyanomethyl, 2-Cyanoethyl oder 1-Cyanoethyl; C₃-C₆-Halogenalkenyl, insbesondere C₃- und C₄-Fluor- oder Chloralkenyl, z.B. 3-Chlorallyl oder 2,3-Dichlorallyl; C₁-C₆-Alkoxycarbonyl-C₁-C₆-alkyl, insbesondere C₁-C₄-Alkoxycarbonyl-C₁-C₆-alkyl wie Methoxycarbonylmethyl, Ethoxycartonylmethyl, Methoxycarbonylethyl oder Ethoxycarbonylethyl, C₁-C₆-Alkoxy- oder C₁-C₆-Alkylthio-C₁-C₆-alkyl, insbesondere C₁-C₄-Alkoxy-C₁-C₄-alkyl oder C₁-C₄-Alkylthio-C₁-C₄-alkyl, z.B. Methoxymethyl, Ethoxymethyl, Methoxyethyl oder Ethoxyethyl und die entsprechenden Alkylthioalkylreste; C₁-C₆-Alkylimino, insbesondere C₁-C₄-Alkylimin, z.B. Ethylimino oder 2-Propylimino; C₁-C₆-Alkyloximino-C₁-C₆-alkyl, insbesondere C₁-C₄-Alkyloximino-C₁-C₄-alkyl, z.B. 2-Propyliminoethyl oder 2-Propyloximino-propyl;
   Phenyl oder Phenyl, das durch ein oder mehrere, insbesondere ein bis drei Reste, ausgewählt aus der Gruppe C₁-C₆-Alkyl, insbesondere Methyl, Ethyl, Propyl; C₃-C₆-Alkenyl, z.B. Allyl, Halogen, z.B. Fluor, Chlor, Brom oder Iod, Cyano, Nitro;
   C₁-C₆-Alkoxy, z.B. Methoxy oder Ethoxy oder C₁-C₆-Alkoxycarbonyl, insbesondere C₁-C₄-Alkoxycarbonyl, z.B. Methoxycarbonyl substituiert ist, Benzyl oder Benzyl, das wie voranstehend definiert an der Phenylgruppe substituiert ist;
(d) NR¹⁰R¹¹, wobei R¹⁰ und R¹¹ folgende Bedeutung haben: Wasserstoff, C₁-C₆-Alkyl-, insbesondere C₁-C₄-Alkyl wie Methyl, Ethyl, n-Propyl, iso-Propyl, n-Butyl, iso-Butyl, tert.-Butyl, sek.-Butyl; C₃-C₆-Alkenyl- oder C₃-C₆-Alkinyl, z.B. Allyl, Propargyl; C₃-C₇-Cycloalkyl wie Cyclopropyl, Cyclopentyl und Cyclohexyl; C₁-C₆-Halogenalkyl, insbesondere C₁-C₄-Halogenalkyl, wobei Halogen vorzugsweise Fluor, Chlor oder Brom bedeutet, z.B. 2,2,2-Trifluorethyl;
   C₁-C₆-Alkylcarbonyl oder C₁-C₆-Alkoxycarbonyl, insbesondere C₁-C₄-Alkylcarbonyl oder C₁-C₄-Alkoxycarbonyl wie Methoxycarbonyl, Ethoxycarbonyl, Methylcarbonyl oder Ethylcarbonyl; C₁-C₆-Alkoxy-C₁-C₆-alkyl, insbesondere C₁-C₄-Alkoxy-C₁-C₄-alkyl, z.B. Methoxymethyl, Ethoxymethyl, n-Propoxymethyl, tert.-Butoxymethyl, 1-Methoxyethyl, 2-Methoxyethyl, 2-Ethoxypropy;
   Phenyl, Phenyl substituiert durch 1 bis 3 Reste ausgewählt aus der Gruppe C₁-C₆-Alkyl, insbesondere Methyl, Ethyl, C₃-C₆-Alkenyl, z.B. Alkyl, Halogen wie Fluor, Chlor, Brom oder Iod, Cyano, Nitro, C₁-C₆-Alkoxy- oder Alkoxycarbonyl-, insbesondere C₁-C₄-Alkoxy oder C₁-C₄-Alkoxycarbonyl, z.B. Methoxy oder Ethoxy, Methoxycarbonyl oder Ethoxycarbonyl;
   R¹⁰ und R¹¹ können auch zusammen mit dem Stickstoffatom an das sie gebunden sind einen gesättigten oder ungesättigten 4- bis 7-gliedrigen, insbesondere 6-gliedrigen Heterocyclus bilden, bei dem ein Ringglied -NH-, -S- oder -O- ist; beispielsweise bilden R¹⁰ und R¹¹ zusammen eine Gruppe -CH₂-CH₂-O-CH₂-CH₂, CH₂-CH₂-S-CH₂-CH₂, CH₂-CH₂-O-CR-CH-.

Im Hinblick auf die Verwendung der erfindungsgemäßen substituierten N-Phenylglutarimide I und der N-Phenylglutaramid-Derivate II als herbizid und defoliant/desikkant wirksame Verbindungen sind die folgenden Bedeutungen der Variable B ganz besonders bevorzugt:

**Tabelle 1**

| Nr. | B | Nr. | B |
|---|---|---|---|
| B.01 | -H | B.39 | -O-(CH₃-phenyl) |
| B.02 | -OH | B.40 | -O-(2,4-Cl₂-Phenyl) |
| B.03 | -OCH₃ | B.41 | -O-(2,4-(CH₃)₂-phenyl-) |
| B.04 | -OC₂H₅ | B.42 | -O-CH₂CN |
| B.05 | -O-n-C₃H₇ | B.43 | -O-CH₂CH=CCl₂ |
| B.06 | -O-i-C₃H₇ | B.44 | -O-CH₂CH=CHCl |
| B.07 | -O-n-C₄H₉ | B.45 | -O-CH₂OCH₃ |
| B.08 | -O-i-C₄H₉ | B.46 | -O-CH₂OC₂H₅ |
| B.09 | -O-s-C₄H₉ | B.47 | -O-C₂H₄OCH₃ |
| B.10 | -O-tert.-C₄H₉ | B.48 | -O-C₂H₄OC₂H₅ |
| B.11 | -O-n-C₅H₁₁ | B.49 | -O-CH(CH₃) -OCH₃ |
| B.12 | -O-n-C₆H₁₃ | B.50 | -O-CH(CH₃)-OC₂H₅ |
| B.13 | -O-CH₂CH=CH₂ | B.51 | -OCH₂C=NOCH₃ |
| B.14 | -O-CH(CH₃)CH=CH₂ | B.52 | -O-C₂H₄C-NOCH₃ |
| B.15 | -O-CH-CH=CH-CH₂ | B.53 | -O-CH₂C=NOC₂H₅ |
| B.16 | -O-CH₂-C≡CH | B.54 | -O-C(O)CH₃ |
| B.17 | -O-CH(CH₃)-C≡CH | B.55 | -O-C(O)C₂H₅ |
| B.18 | -O-CH₂-C≡C-CH₃ | B.56 | -O-C₂H₄C=NOC₂H₅ |
| B.19 | -O-cyclopropyl | B.57 | -SCH₃ |
| B.20 | -O-cyclobutyl | B.58 | -SC₂H₅ |
| B.21 | -O-cyclopentyl | B.59 | -S-n-C₃H₇ |
| B.22 | -O-cyclohexyl | B.60 | -S-i-C₃H₇ |
| B.23 | -O-CH₂-CF₃ | B.61 | -S-CH₂CH=CH₂ |
| B.24 | -O-CH₂-CCl₃ | B.62 | -S-CH₂C≡CH |
| B.25 | -O-(CH₂)₃-Br | B.63 | -S-phenyl |
| B.26 | -O-phenyl | B.64 | -S-CH₂CN |
| B.27 | -O-(2-F-phenyl) | B.65 | -S-CH₂OCH₃ |
| B.28 | -O-(2-Cl-phenyl) | B.66 | -CH₃ |
| B.29 | -O-(2-Br-phenyl) | B.67 | -C₂H₇ |
| B.30 | -O-(3-F-phenyl) | B.68 | -n-C₃H₇ |
| B.31 | -O-(3-Cl-phenyl) | B.69 | -i-C₃H₇ |
| B.32 | -O-(3-Br-phenyl) | B.70 | -n-C₄H₉ |
| B.33 | -O-(4-F-phenyl) | B.71 | -i-C₄H₉ |
| B.34 | -O-(4-Cl-phenyl) | B.72 | -s-C₄H₉ |
| B.35 | -O-(4-Br-phenyl) | B.73 | -tert.-C₄H₉ |
| B.36 | -O-(4-OCH₃-phenyl) | B.74 | -n-C₅H₁₁ |
| B.37 | -O-(4-CN-phenyl) | B.75 | -n-C₆H₁₃ |
| B.38 | -O-(4-COOCH₃-phenyl) | B.76 | -CH₂CH=CH₂ |
| B.77 | -CH₂C≡CH | B.108 | -NH-CH(CH₃)-CH=CH₂ |
| B.78 | -CH(CH₃)CH=CH₂ | B.109 | -NH-CH₂-C≡CH |
| B.79 | -CH(CH₃C≡CH | B.110 | -NH-CH(CH₃)-C≡CH |
| B.80 | -CH₂Cl | B.111 | -N(CH₃)-CH₂CH≡CH |
| B.81 | -CH₂Br | B.112 | -N(CH₃)-CH₂C=CH |
| B.82 | -CHCl₂ | B.113 | -NH-cyclopropyl |
| B.83 | -CF₃ | B.114 | -NH-cyclobutyl |
| B.84 | -Cyclopropyl | B.115 | -NH-cyclopentyl |
| B.85 | -Cyclobutyl | B.116 | -NH-cyclohexyl |
| B.86 | -Cyclopentyl | B.117 | -N(CH3)-cyclohexyl |
| B.87 | -Cyclohexyl | B.118 | -N(C₂H₅)-cyclohexyl |
| B.88 | -Phenyl | B.119 | -NH-COCH₃ |
| B.89 | 2-F-phenyl | B.120 | -NH-COC₂H₅ |
| B.90 | 3-F-phenyl | B.121 | -NH-COOCH₃ |
| B.91 | 4-F-phenyl | B.122 | -NH-CH₂OCH₃ |
| B.92 | 2-Cl-phenyl | B.123 | -NH-(CH₂)₂CCH₃ |
| B.93 | 4-Cl-phenyl | B.124 | -N-piperindinyl |
| B.94 | 2,4-C1₂-phenyl | B.125 | -N-pyrrolidinyl |
| B.95 | -CH₂-OCH₃ | B.126 | -N-morpholin-4-yl |
| B.96 | -CH(OCH₃)₂ | B.127 | -N-piperazinyl |
| B.97 | -CH₂-SCH₃ | B.128 | -NH-phenyl |
| B.98 | -NH₂ | B.129 | -NH-(2-CH₃-phenyl) |
| B.99 | -NHCH₃ | B.130 | -NH-(2-F-phenyl) |
| B.100 | -NH-n-C₃H₇ | B.131 | -NH-(4-F-phenyl) |
| B.101 | -NH-i-C₃H₇ | B.132 | -NH-(2-Cl-phenyl) |
| B.102 | -NH-n-C₄H₉ | B.133 | -NH-(4-Cl-phenyl) |
| B.103 | -N(CH₃)₂ | B.134 | -NH-(2,4-Cl₂-phenyl) |
| B.104 | -N(C₂H₅)₂ | B.135 | -O-CO-OCH₃ |
| B.105 | -N(CH₃)C₂H₅ | B.136 | -O-CO-OC₂H₅ |
| B.106 | -N(n-C₃H₇)₂ | B.137 | -O-CH₂-COOCH₃ |
| B.107 | -NH-CH₂CH=CH₂ | B.138 | -O-CH(CH₃)-COOCH₃ |

Die Verbindungen der Formel I können in Form ihrer landwirtschaftlich brauchbaren Salze vorliegen, wobei es auf die Art des Salzes im allgemeinen nicht ankommt. Üblicherweise kommen die Salze von solchen Basen in Betracht, welche die herbizide Wirkung von I nicht negativ beeinträchtigen.

In Betracht kommen beispielsweise Alkalimetallsalze, insbesondere Natrium- und Kaliumsalze, Erdalkalimetallsalze wie insbesondere Calcium-, magnesium- und Bariumsalze, Mangan-, Kupfer-, Zink- oder Eisensalze sowie Ammoniumsalze wie Tetraalkyl- und Benzyltrialkylammoniumsalze, Phosphonium-, Sulfoniumsalze wie Trialkylsulfoniumsalze oder Sulfoxoniumsalze.

Die substituierten N-Phenylglutarimide der Formel I sind auf verschiedene Weise erhältlich und zwar vorzugsweise nach einem der folgenden Verfahren:
a) mit X¹ - O, S und X²= O, durch Cyclisierung eines N-Phenylglutarsäureamids der Formel II 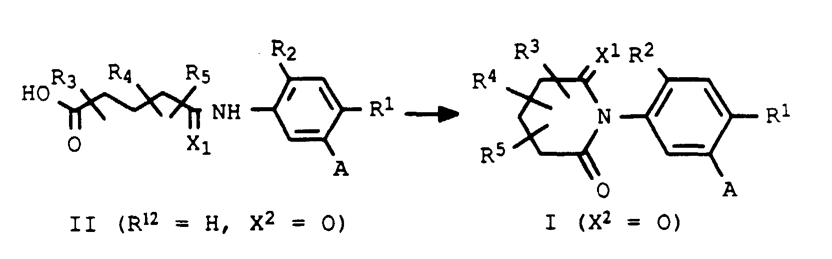
   Die Reaktion wird üblicherweise in einem inerten Lösungsmittel und in Gegenwart eines Dehydratisierungsmittels durchgeführt.
   Als Lösungsmittel werden inerte organische Lösungsmittel wie Kohlenwasserstoffe, z.B. n-Hexan, Ligroin oder Petrolether; Aromaten, z.B. Toluol oder Xylol; halogenierte Kohlenwasserstoffe, z.B. Dichlormethan, Trichlormethan oder Chlorbenzol; Ether, z.B. 1,2-Dimethoxyethan, Diethylether, Tetrahydrofuran oder Dioxan; Ketone wie Aceton und tert.-Butylmethylketon; Alkohole wie Methanol, Ethanol und Isopropanol; Nitrile wie Acetonitril; polare organische Lösungsmittel wie Dimethylformamid und Dimethylsulfoxid; organische Säuren, z.B. Essigsäure oder Trifluoressigsäure; Wasser oder Gemische dieser Lösungsmittel verwendet.
   Als Dehydratisierungsreagenz bieten sich z.B. Säurehalogenide, z.B. Acetylchlorid, Propionylchlorid, Thionylchlorid, Phosphoroxychlorid oder Phosphorpentachlorid; Anhydride, wie Essigsäureanhydrid, oder Säuren, z.B. Phosphorsäure, Polyphosphorsäure oder Schwefelsäure, an.
   Das Verhältnis Dehydratisierungsmittel/Edukt II ist nicht kritisch. Üblicherweise werden 0,5 bis 1,5 Äquivalente, bevorzugt 0,7 bis 1,2 Äquivalente, verwendet. In einigen Fällen kann es jedoch auch sinnvoll sein, das Dehydratisierungsmittel in einem großen Überschuß und damit gleichzeitig als Lösungsmittel zu verwenden.
   Die Reaktionstemperatur liegt zwischen etwa 0°C und 150°C, vorzugsweise zwischen 35°C und der Rückflußtemperatur des verwendeten Lösungsmittels.
   In einigen Fällen kann es vorteilhaft sein, das bei der Reaktion entstehende Wasser mit physikalischen Mitteln, wie z.B. der azeotropen Destillation, der Wasserauskreisung oder dem Entwässern mittels Molekularsieb aus der Reaktion zu entfernen, so daß in einigen Fällen auf ein chemisches Entwässerungsmittel verzichtet werden kann.
   Normalerweise arbeitet man bei Atmosphärendruck oder unter dem Eigendruck des jeweiligen Lösungsmittels. Höherer oder niedriger Druck ist möglich, bringt aber im allgemeinen keine Vorteile.
b) mit X¹ = O, S und X² = O, durch Cyclisierung eines N-Phenylglutarsäureamids der Formel II 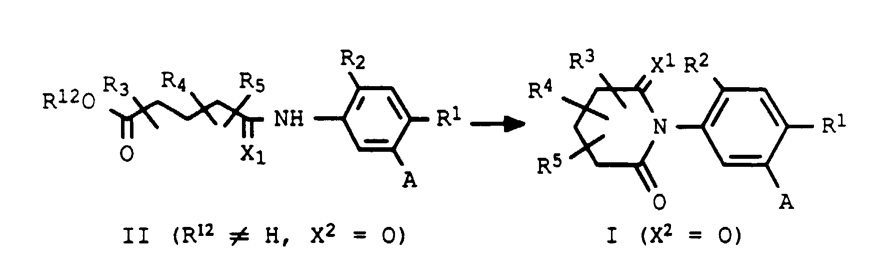 in einem inerten Lösungsmittel und in Gegenwart einer Säure oder einer Base.
   Als Lösungsmittel kommen Kohlenwasserstoffe, z.B. n-Hexan, Ligroin oder Petrolether; Aromaten wie Toluol und Xylol; halogenierte Kohlenwasserstoffe, z.B. Dichlormethan, Trichlormethan oder Chlorbenzol; organische Stickstoffbasen, z.B. Triethylamin oder Pyridin; Ether wie 1,2-Dimethoxyethan, Diethylether, Tetrahydrofuran und Dioxan; Ketone wie Aceton und tert.-Butylmethylketon; Alkohole wie Methanol, Ethanol und Isopropanol; Nitrile wie Acetonitril; polare organische Lösungsmittel, z.B. Dimethylformamid, N-Methylpyrrolidon oder Dimethylsulfoxid; organische Säuren, z.B. Essigsäure oder Trifluoressigsäure; Wasser oder Gemische dieser Lösungsmittel in Betracht.
   Als Base verwendet man üblicherweise organische Stickstoffbasen, z.B. Pyridin, Triethylamin oder 1,4-Diaza-bicyclo-[2,2,2]octan; oder anorganische Basen, z.B. Natriumhydrid, Natriumhydroxid, Kaliumhydroxid, Natriumcarbonat oder Kaliumcarbonat; Alkalimetallalkoholate wie Natriummethylat, Natriumethylat und Kalium-tert.-butylat.
   Als Säure eignen sich z.B. organische Säuren wie p-Toluolsulfonsäure und Essigsäure, oder Mineralsäuren wie Schwefelsäure und Phosphorsäure, wobei die Säuren gleichzeitig auch als Lösungs- bzw. Verdünnungsmittel verwendet werden können.
   Die Reaktionstemperatur liegt zwischen 20°C und 200°C, vorzugsweise zwischen 50°C und der Rückflußtemperatur des verwendeten Lösungsmittels.
   Bezüglich des Druck gelten die Angaben für Methode a).
c) mit X¹ = S und X² = O oder S, durch Umsetzung eines substituierten N-Phenylglutarimids der Formel I, wobei X¹ Sauerstoff bedeutet, mit einem geeigneten Schwefelungsreagenz 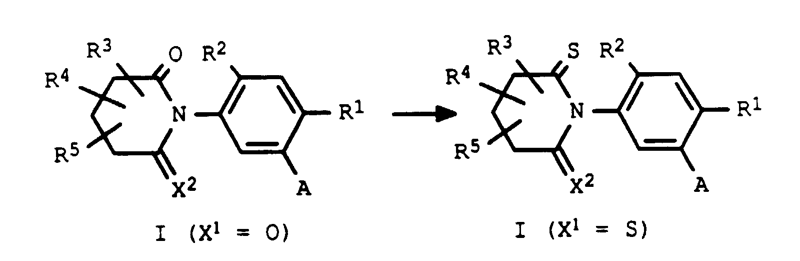
   Die Umsetzung erfolgt in der Regel in einem inerten Lösungsmittel. Als Lösungsmittel kommen z.B. Aromaten, wie Toluol oder Xylol, chlorierte Kohlenwaserstoffe, z.B. Dichlormethan, Chloroform oder Chlorbenzol; Ether wie Dialkylether, 1,2-Dimethoxyethan, Tetrahydrofuran und Dioxan; Alkohole wie Methanol und Ethanol; Ketone wie Aceton, oder Wasser, sowie ein Gemisch dieser Lösungsmittel in Betracht.
   Als Schwefelungsreagenz eignen sich besonders gut Phosphor(V)-sulfid und 2,4-Bis-(4-methoxyphenyl)-1,2,3,4-dithiadiphosphetan-2,4-dithion ("Lawesson-Reagenz").
   Die Menge an Schwefelungsreagenz ist nicht kritisch, normalerweise verwendet man die 1- bis 5-fache Molmenge, bezogen auf das zu schwefelnde N-Phenyl-substituierte Glutarimid, sofern man beide Gruppen X¹ und X² = O durch Schwefel ersetzen möchte. Will man nur einen Rest X ersetzen, so wählt man vorteilhaft etwa äquimolare Mengen.
   Normalerweise liegt die Reaktionstemperatur zwischen 20°C und 200°C, vorzugsweise zwischen 40°C und der Rückflußtemperatur des verwendeten Lösungsmittels.
   Wenn das zu schwefelnde Glutarimid der Formel I eine weitere gegenüber dem Schwefelungsreagenz reaktivere Carbonylgruppe als die Imidgruppe besitzt, z.B. wenn B Wasserstoff oder Alkyl bedeutet, so läßt sich die Selektivität der Schwefelungsreaktion durch Schutzgruppentechnik gewährleisten, indem man beispielsweise die Carbonylgruppe in der Seitenkette A durch eine übliche Schutzgruppe blockiert. Als Schutzgruppe eignen sich z.B. Acetale oder andere dem Fachmann wohl bekannte Gruppen, wie sie z.B. in "Protective Groups in Organic Synthesis", Th.W. Greene, Wiley & Sons, New York 1981 beschrieben sind.
   Im allgemeinen sind die substituierten N-Phenylglutarimide I nach einem der vorstehend genannten Syntheseverfahren herstellbar. Aus wirtschaftlichen oder verfahrenstechnischen Gründen kann es jedoch zweckmäßiger sein, einige Verbindungen I aus ähnlichen substituierten N-Phenylglutarimiden I, die sich jedoch insbesondere in der Bedeutung der Reste A oder B unterscheiden, herzustellen, und zwar auf an sich bekannte Weise, z.B. durch Esterhydrolyse, Veresterung, Amidierung, Acetalisierung, Acetalhydrolyse, Kondensationsreaktion, Wittig-Reaktion, Peterson-Olefinierung, Veretherung, Alkylierung, Oxidation oder Reduktion.
   Die substituierten N-Phenylglutarimide I können bei den vorstehend beschriebenen Verfahren als Isomerengemische erhalten werden. Die erhaltenen Isomere lassen sich jedoch gewünschtenfalls mit den hierfür üblichen Methoden, z.B. durch Kristallisation, Chromatographie (LC, HPLC, etc.), gegebenenfalls an einem optisch aktiven Absorbat, in die reinen Isomeren trennen.
   Die N-Phenylglutarsäureamide der Formel II sind neu. Sie sind wertvolle Zwischenverbindungen zur Herstellung der erfindungsgemäßen Endprodukte der Formel I. Die Erfindung betrifft somit auch neue Glutarsäureamide bzw. Glutaresteramide (Formel II), worin die Reste R¹ bis R⁸, X¹, X²_{,} A und B wie unter Formel I definiert sind und der Rest R⁹ H, C₁-C₆-Alkyl oder Benzyl bedeutet.
   Die Verbindungen II zeigen überraschenderweise zudem auch noch herbizide Aktivität und defoliante/desikkante Wirkung.
   Die N-Phenylglutarsäureamide II sind auf verschiedene Weise erhältlich und zwar vorzugsweise nach einem der folgenden Verfahren:
d) durch Umsetzung einer Anilins der Formel III mit einem Glutarsäureanhydrid der Formel IV 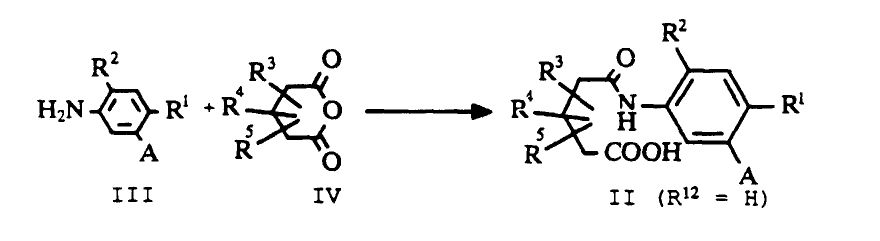
   Geeignete Lösungsmittel für diese Reaktion sind z.B. Ether wie Diethylether, Tetrahydrofuran und Dioxan; Kohlenwasserstoffe wie Hexan, Petrolether, Toluol und Xylol; Acetonitril; Amide wie Dimethylformamid und chlorierte Kohlenwasserstoffe wie Dichlormethan, Chloroform und Chlorbenzol, sowie Mischungen der genannten Lösungsmittel.
   Man arbeitet üblicherweise in einem Temperaturbereich von (-20) bis 100°C, vorzugsweise 0 bis 70°C.
   Zweckmäßigerweise arbeitet man bei Atmosphärendruck oder unter dem Eigendruck des jeweiligen Lösungsmittels. Höherer oder niedrigerer Druck ist möglich, bringt im allgemeinen aber keine Vorteile.
e) durch Umsetzung eines Anilins der Formel III mit einem Glutarsäuremonoester der Formel V in Gegenwart einer Base. 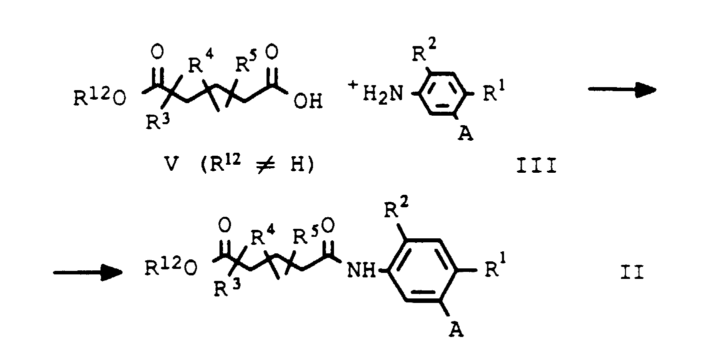
   Als Lösungsmittel für diese Reaktion eignen sich z.B. Kohlenwasserstoffe wie Hexan, Petrolether, Benzol, Toluol und Xylol; halogenierte Kohlenwasserstoffe wie Dichlormethan, Chloroform, Tetrachlorkohlenwasserstoff und Chlorbenzol; Ether wie Diethylether, tert.-Butylmethylether, Tetrahydrofuran und Dioxan; Ketone wie Aceton; Nitrile wie Acetonitril; tertiäre Amine wie Pyridin und N,N-Diethylanilin; Säureamide wie Dimethylformamid und N-Methylpyrrolidon; organische Schwefelverbindungen wie Dimethylsulfoxid und Sulfolan; des weiteren Wasser oder Mischungen dieser Lösungsmittel.
   Als Base eignen sich z.B. organische Stickstoffbasen wie Pyridin, Triethylamin und 1,4-Diazabicyclo-[2,2,2]-octan, anorganische Basen wie Natriumhydrid, Kaliumhydrid, Kaliumhydroxid, Natriumhydroxid, Natriumcarbonat und Kaliumcarbonat; Metallalkoholate wie Natriummethanolat, Natriumethanolat und Kalium-tert.-butanolat.
   Das Verhältnis der Base zu III ist üblicherweise 0,5 bis 1,5, bevorzugt 0,7 bis 1,2.
   In einer bevorzugten Ausführungsform wird die Säure V vor der Umsetzung mit III in ein aktiviertes Säurederivat, beispielsweise ein Halogenid, bevorzugt Chlorid, ein Imidazolid oder ein gemischtes Anhydrid, z.B. mit Ethylkohlensäure, überführt. Geeignete Reagenzien hierzu sind z.B. Thionylchlorid, Phosphoroxychlorid, Phosgen oder Imidazol oder Chlorameisensäureethylester.
   In einigen Fällen läßt sich statt einer Base auch ein Kondensationsreagenz, z.B. Dicyclohexylcarbodiimid, verwenden.
   Die Reaktionstemperatur liegt zwischen (-40) und 160°C, bevorzugt (-20) und 130°C.
   Das Verhältnis der Ausgangsverbindungen III und IV ist nicht kritisch, bevorzugt werden etwa äquimolare Mengen beider Edukte umgesetzt.
   Bezüglich des Druckes gelten die Angaben für Methode d).
f) durch Umsetzung einer Verbindung der Formel II, wobei X¹ Sauerstoff bedeutet, mit einem geeigneten Schwefelungsreagenz 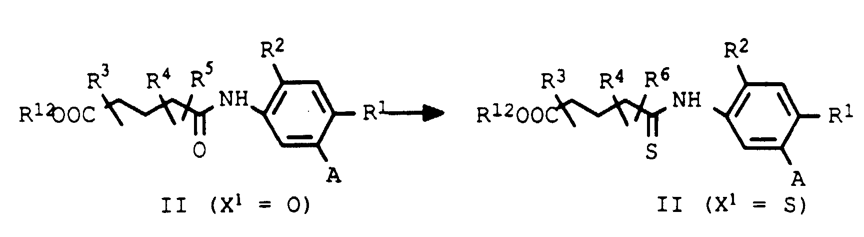
   Die Reaktionsbedingungen, Lösungsmittel etc. sind bereits oben für I (Verfahren c)) beschrieben und können analog auf II übertragen werden.
   Bezüglich des Druckes gelten die Angaben für Methode d).
   Eine weitere erfindungsgemäße Herstellungsweise der substituierten N-Phenylglutarimide I ist die direkte Darstellung aus dem Anilin III ohne Isolierung der Zwischenprodukte II.
   Die Ausgangsmaterialien III, IV und V sind bekannt oder nach an sich bekannten Verfahren, z.B. wie in EP-A-391 847, 415 642, 454 444, DE-A-3 941 562, DE-A-40 42 194, EP-A-400 427 oder EP-A-415 641 beschrieben, herstellbar.

Unter den substituierten N-Phenylglutarsäureimiden sind die Verbindungen I' 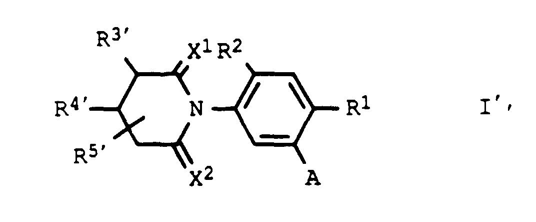 wobei R⁴' C₁-C₆-Halogenalkyl, R³' und R⁵'unabhängig voneinander Wasserstoff, C₁-C₆-Alkyl oder C₁-C₆-Halogenalkyl bedeuten, besonders bevorzugt.

Im Hinblick auf die herbizide Verwendung ganz besonders bevorzugte Verbindungen I' sind in nachfolgender Tabelle 2 aufgeführt.

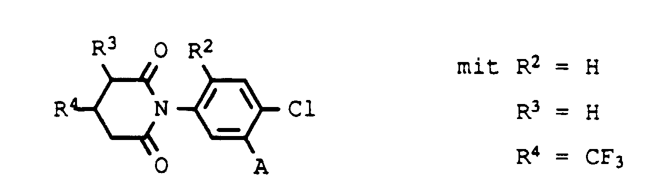

**Tabelle 2**

| Nr. | A |
|---|---|
| Ia1 | CH₂-CHCl-COOCH₃ |
| Ia2 | CH₂-CHCl-COOC₂H₅ |
| Ia3 | CH₂-CHBr-COOCH₃ |
| Ia4 | CH₂-CHBr-COOC₂H₅ |
| Ia5 | CH₂-CHI-COOCH₃ |
| Ia6 | CH₂-CHI-COOC₂H₅ |
| Ia7 | CH=CH-COOCH₃ |
| Ia8 | CH=CH-COOC₂H₅ |
| Ia9 | CH=CCl-COOCH₃ |
| Ia10 | CH=CCl-COOC₂H₅ |
| Ia11 | CH=CBr-COOCH₃ |
| Ia12 | CH=CBr-COOC₂H₅ |
| Ia13 | CH=Cl-COOCH₃ |
| Ia14 | CH=Cl-COOC₂H₅ |
| Ia15 | CH=CF-COOCH₃ |
| Ia16 | CH=CF-COOC₂H₅ |
| Ia17 | CH=C(CN)-COOCH₃ |
| Ia18 | CH=C(CN)-COOC₂H₅ |
| Ia19 | CH=C(COOCH₃)₂ |
| Ia20 | CH=C(COOC₂H₅)₂ |
| Ia21 | CH=C(COOCH₃)-COOC₂H₅ |
| Ia22 | CH=C(CH₃)-COOCH₃ |
| Ia23 | CH=C(CH₃)-COOC₂H₅ |
| Ia24 | CH=C(C₂H₅)-COOC₂H₅ |
| Ia25 | CH=C(CF₃)-COOCH₃ |
| Ia26 | CH=C(CF₃)-COOC₂H₅ |
| Ia27 | CH=C(COCH₃)-COOCH₃ |
| Ia28 | CH=C(COCH₃)-COOC₂H₅ |
| Ia29 | CH=CH-CO-CH₃ |
| Ia30 | CH=CH-CO-CH₂Cl |
| Ia31 | CH=CH-CO-CHCl₂ |
| Ia32 | CH=CH-CO-CH₂OCH₃ |
| Ia33 | CH=CCl-COCH₃ |
| Ia34 | CH=CBr-COCH₃ |
| Ia35 | CH=C(CH₃)-CHO |
| Ia36 | CH=C(CH₃)-CO-CH₃ |
| Ia37 | CH=CH-COO(CH₂)₂OCH₃ |
| Ia38 | CH=CH-COO (CH₂)₂OC₂H₅ |
| Ia39 | CH=CCl-COO(CH₂)₂OCH₃ |
| Ia40 | CH=CCl-COO(CH₂)₂OC₂H₅ |
| Ia41 | CH=CBr-COO(CH₂)₂OCH₃ |
| Ia42 | CH=CBr-COO(CH₂)₂OC₂H₅ |
| Ia43 | CH=CH-COOCH₂CF₃ |
| Ia44 | CH=CCl-COOCH₂CF₃ |
| Ia45 | CH=CBr-COOCH₂CF₃ |
| Ia46 | CH=Cl-COOCH₂CF₃ |
| Ia47 | CH=CF-COOCH₂CF₃ |
| Ia48 | CH=CH-COO-N=C(CH₃)₂ |
| Ia49 | CH=CCl-COO-N=C(CH₃)₂ |
| Ia50 | CH=CBr-COO-N=C(CH₃)₂ |

Des weiteren sind folgende substituierte N-Phenylglutarimide I besonders bevorzugt:
- die Verbindungen Ib1 bis Ib50, die sich von den Verbindungen Ia1 bis Ia50 dadurch unterscheiden, daß R² Fluor bedeutet; 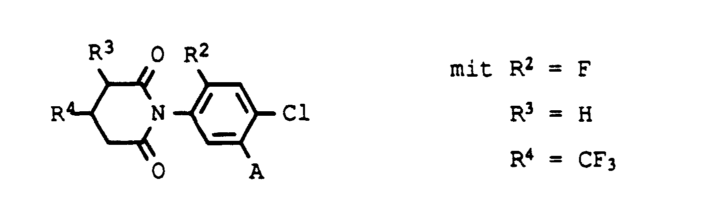
- die Verbindungen Ic1 bis Ic50, die sich von den Verbindungen Ia1 bis Ia50 dadurch unterscheiden, daß R³ Methyl bedeutet; 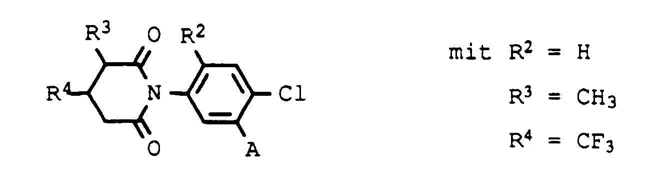
- die Verbindungen Id1 bis Id50, die sich von den Verbindungen Ia50 bis Ia50 dadurch unterscheiden, daß R² Fluor und R³ Methyl bedeutet; 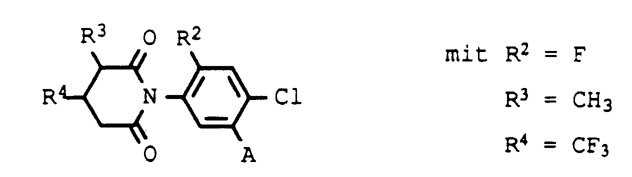
- die Verbindungen Ie1 bis Ie50, die sich von den Verbindungen Ia1 bis Ia50 dadurch unterscheiden, daß R⁴ Difluormethyl bedeutet; 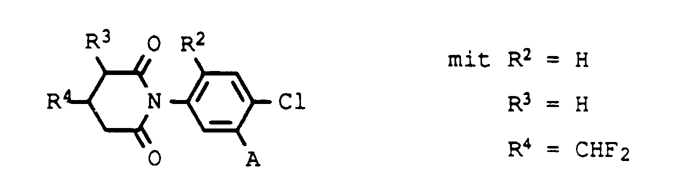
- die Verbindungen If1 bis If50, die sich von den Verbindungen Ia1 bis Ia50 dadurch unterscheiden, daß R² Fluor und R⁴ Difluormethyl bedeutet; 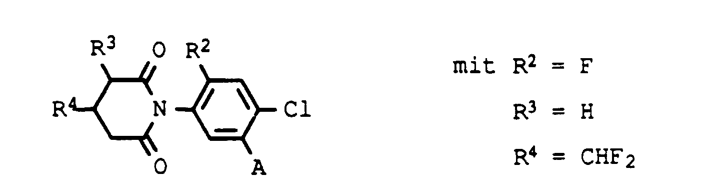
- die Verbindungen Ig1 bis Ig50, die sich von den Verbindungen Ia1 bis Ia50 dadurch unterscheiden, daß R³ Methyl und R⁴ Difluormethyl bedeutet; 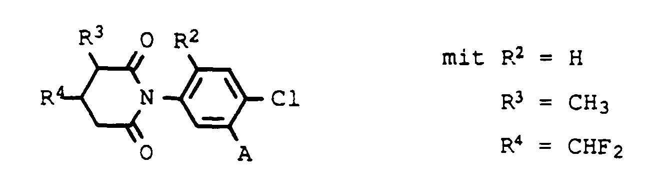
- die Verbindungen Ih1 bis Ih50, die sich von den Verbindungen Ia1 bis Ia50 dadurch unterscheiden, daß R² Fluor, R³ Methyl und R⁴ Difluormethyl bedeutet.

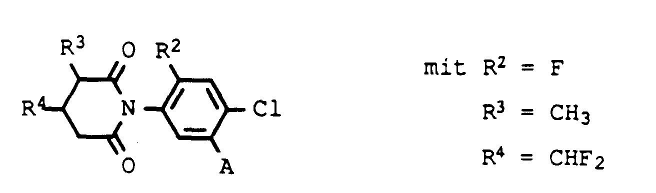

Die erfindungsgemäßen substituierten N-Phenylglutarimide I bzw. N-Phenylglutarsäureamide II sowie deren landwirtschaftlich brauchbaren Salze eignen sich als Herbizide, insbesondere zur Bekämpfung von dikotylen Unkräutern und als Defoliations-/Desikkationsmittel, insbesondere zur Entlaubung von Baumwolle, und als Defoliantien zur Austrocknung der oberirdischen Pflanzenteile bei Kulturpflanzen, z.B. Kartoffel, Sonnenblume, Sojabohne und Raps. Damit wird ein vollständig mechanisiertes Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Außerdem führen sie zu einer gleichmäßigen Abreife der Erntefrüchte.

Derselbe Mechanismus, d.h. die Förderung der Ausbildung von Trenngewebe zwischen Frucht- bzw. Blatt- und Sproßteil der Pflanze ist auch für ein gut kontrollierbares Entblättern von Nutzpflanzen wie insbesondere Baumwolle wesentlich. Außerdem führt die Verkürzung des Zeitintervalls, in dem die einzelnen Baumwollpflanzen reif werden, zu einer erhöhten Faserqualität nach der Ernte.

Die Verbindungen I und II bzw. die sie enthaltenden herbiziden oder Desikkations-/Defoliations-Mittel können beispielsweise in Form von direkt versprühbaren wässrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der Wirkstoffe gewährleisten.

Die Verbindungen I und II eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethyler- oder Polyoxypropylen-alkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Beispiele für solche Zubereitungen sind:
I. eine Mischung aus 20 Gew.-Teilen der Verbindung Nr. 1.2, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes und 40 Mol Ethylenoxid an 1 Mol Ricinusöl. Durch feines Verteilen des Gemisches in 100 000 Gew.-Teilen Wasser erhält man eine wäßrige Dispersion, die 0,02 Gew.-% des Wirkstoffs enthält.
II. eine Dispersion von 20 Gew.-Teilen der Verbindung Nr. 1.2 in einer Mischung aus 40 Gew.-Teilen Cyclohexanon, 30 Gew.-Teilen Isobutanol, 20 Gew.-Teilen des Anlagerungsproduktes von 7 mol Ethylenoxid an 1 mol Isooctylphenol und 10 Gew.-Teilen des Anlagerungsproduktes von 40 mol Ethylenoxid an 1 mol Ricinusöl. Die Mischung dieser Dispersion mit 100 000 Gewichtsteilen Wasser enthält 0,02 Gew.-% des Wirkstoffes.
III. eine Dispersion von 20 Gew.-Teilen der Verbindung Nr. 1.5 in einer Mischung aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl. Die Mischung dieser Dispersion mit 100 000 Gew.-Teilen Wasser enthält 0,02 % des Wirkstoffes;
IV. eine in einer Hammermühle vermahlene Mischung aus 20 Gew.-Teilen der Verbindung Nr. 1.1, 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel. Durch feines Verteilen der Mischung in 20 000 Gew.-Teilen Wasser erhält man eine Spritzbrühe, die 0,1 Gew.-% des Wirkstoffs enthält;
V. eine Mischung aus 3 Gew.-Teilen der Verbindung Nr. 1.2 und 97 Gew.-Teilen feinteiligem Kaolin. Dieses Stäubemittel enthält 3 Gew.-% Wirkstoff;
VI. eine stabile ölige Dispersion aus 20 Gew.-Teilen der Verbindung Nr. 1.4, 2 Gew.-Teilen des Calciumsalzes der Dodecylbenzolsulfonsäure, 8 Gew.-Teilen Fettalkohol-polyglykolether, 2 Gew.-Teilen des Natriumsalzes eines Phenolsulfonsäure-harnstoffformaldehyd Kondensates und 68 Gew.-Teilen eines paraffinischen Mineralöls;

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1 kg/ha aktive Substanz (a.S.).

In Anbetracht der Vielseitigkeit der Applikationsmethoden können die Verbindungen I und II bzw. sie enthaltende Mittel noch in einer weiteren Zahl von Kulturpflanzen zur Beseitigung unerwünschter Pflanzen eingesetzt werden. In Betracht kommen beispielsweise folgende Kulturen (botanische Namen):

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N. rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Zur Verbreiterung des Wirkungsspektrums und zur Erzielung synergistischer Effekte können die Verbindungen I und II mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I und II - allein oder in Kombination mit anderen Herbiziden - auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können aber auch nichtphytotoxische Öle und ölkonzentrate zugesetzt werden.

### Herstellungsbeispiele

### Beispiel 1

### N- [4-Chlor-3-(2-Chlor-2-ethoxycarbonyl-ethenyl)-phenyl)-3-methylglutarsäuremonoamid (Verbindung 2.1)

Zu einer Lösung von 2,56 g 3-Methylglutarsäureanhydrid in 50 ml Dichlormethan wurden bei 25°C 5,2 g 4-Chlor-3-(2-Chlor-2-ethoxycarbonyl-ethenyl)-anilin in 30 ml Dichlormethan getropft. Nach 3 h wurde der ausgefallene Niederschlag abgetrennt und mit Dichlormethan nachgewaschen. Ausbeute: 5 g; Smp.: 137 - 138°C.

### Beispiel 2

### N- [4-Chlor-3-(2-chlor-2-ethoxycarbonyl-ethenyl)-phenyl]-3-methylglutarsäureimid (Verbindung 1.1)

Eine Lösung von 2,8 g N-[4-Chlor-3-(2-chlor-2-ethoxycarbonylethenyl) -phenyl] -3-methyl-glutarsäuremonoamid und 0,1 g Natriumacetat in 50 ml Acetanhydrid wurde 2 h bei 95°C gerührt. Danach wurde das Lösungsmittel entfernt. Nach Lösen des Rückstandes in 150 ml Dichlormethan wurde mit Natriumhydrogencarbonatlösung, Wasser und Kochsalzlösung gewaschen, getrocknet und im Vakuum eingeengt. Der verbleibende Rückstand wurde mit Petrolether kristallisiert, die Kristalle abgetrennt und getrocknet. Ausbeute: 2,1 g;
Smp.: 82 - 84°C.

### Beispiel 3

### 1- [4-Chlor-3-(2-chlor-2-ethoxycarbonyl-ethenyl)-phenyl]-3-trifluormethyl-6-thiono-(2H)-3,4,5,6-tetrahydropyrid-2-on (Verbindung 1.7)

Zu einer Lösung von 2,1 g N-[4-Chlor-3-(2-chlor-2-ethoxycarbonyl-ethenyl)-phenyl]-3-trifluormethyl-glutarsäureimid in 100 ml Toluol wurden 1,6 g Lawesson-Reagenz* ) gegeben. Die Mischung wurde 4 Std. auf Rückflußtemperatur erhitzt und anschließend mit weiteren 0,8 g Lawesson-Reagenz*)) versetzt. Nach 3 Std. Rühren bei Rückflußtemperatur destillierte man das Lösungsmittel ab. Der Rückstand wurde chromatographisch an Kieselgel (Laufmittel: Toluol) gereinigt. Ausbeute: 0,6 g Öl.
*) 2,4-Bis (4-methoxyphenyl)-1,3,2,4-dithiadiphosphetan-2,4-dion

### Beispiel 4

### N-[4-Chlor-3-(2-chlor-2-ethoxycarbonyl-ethenyl)-phenyl]-3-trifluormethyl-glutarsäureethylesteramid

Zu einer Lösung von 4,2 g (10 mmol) N-[4-Chlor-3-(2-chlor-2-ethoxycarbonyl-ethenyl)-phenyl]-3-trifluormethyl-glutarsäureimid in 100 ml Ethanol wurden bei Raumtemperatur portionsweise 0,13 g (34 mmol) Natriumborhydrid gegeben. Nach 3 Std. Rühren wurde das Reaktionsgemisch in 100 ml Wasser gegeben, wonach man die wäßrige Phase zweimal mit Dichlormethan extrahierte. Die vereinigten org. Phasen wurden über Natriumsulfat getrocknet und eingeengt. Schließlich wurde der Rückstand durch verrühren mit Petrolether gereinigt. Ausbeute: 2 g (42 %); Smp.: 115-117°C.

In analoger Weise wurden die in nachstehenden Tabellen 3 und 4 aufgeführten Wirkstoffe erhalten.

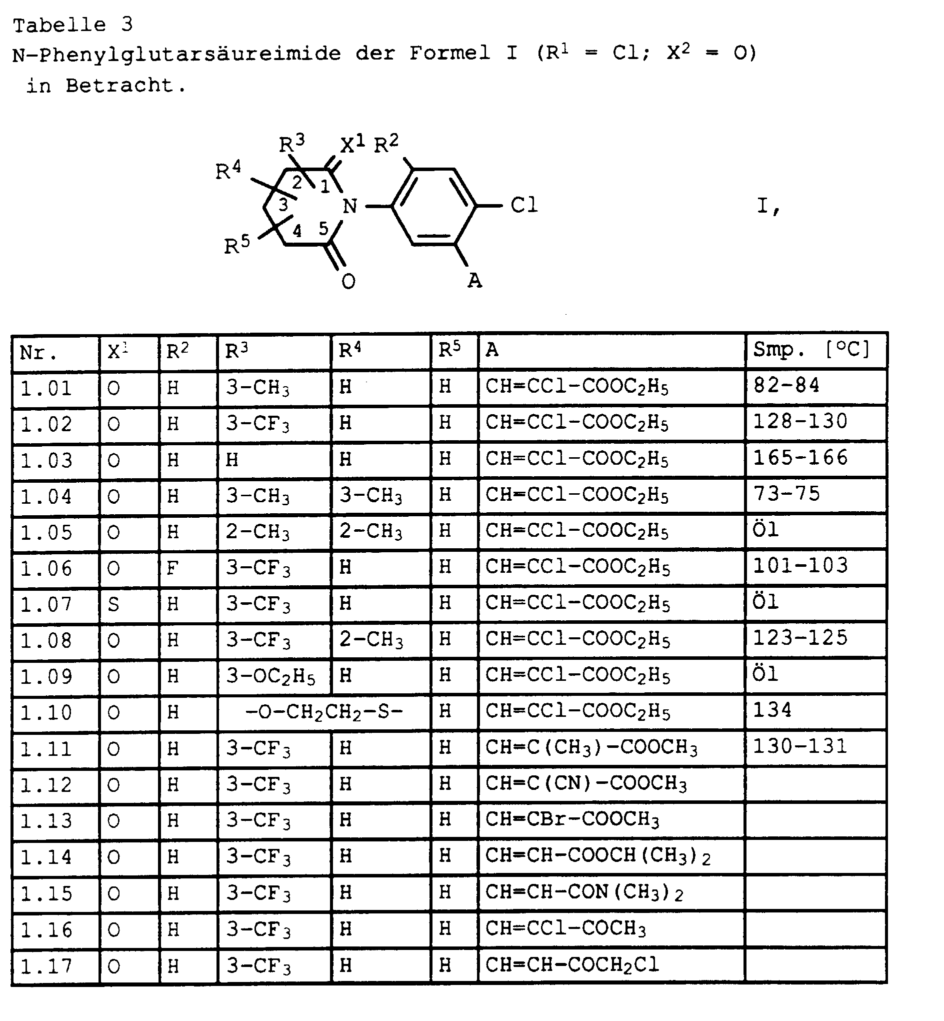

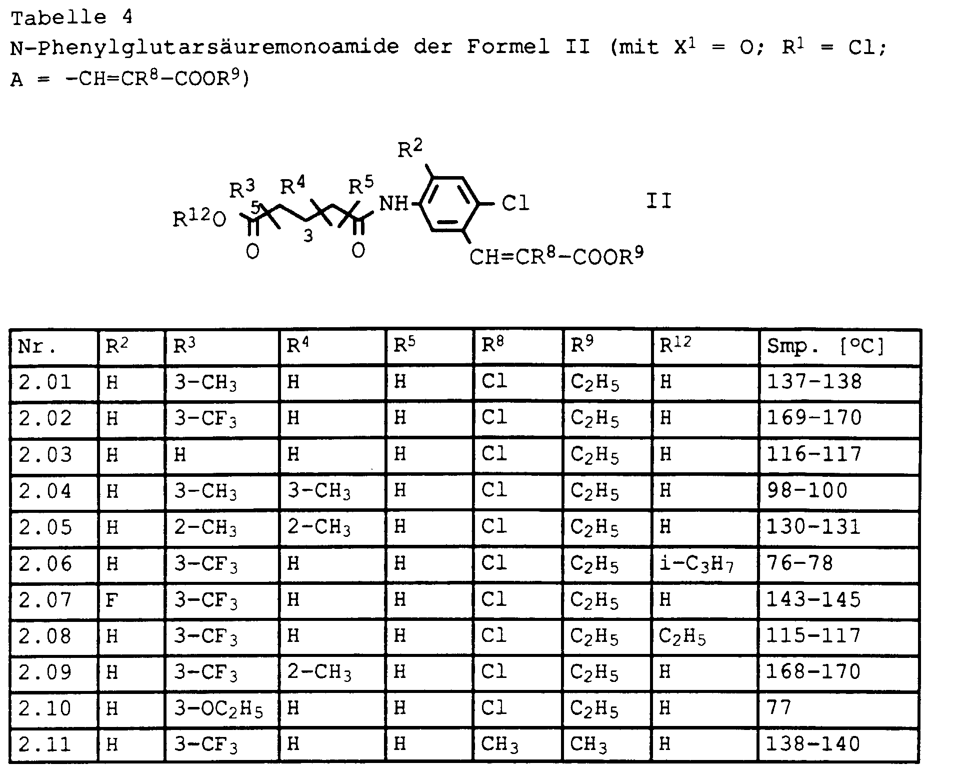

### Anwendungsbeispiele

Die herbizide Wirkung der substituierten N-Phenylglutarsäureimide I und N-Phenylglutarsäureamide II ließ sich durch Gewächshausversuche zeigen:

Als Kulturgefäße dienten Plastikblumentöpfe mit lehmigem Sand mit etwa 3,0 Gew.-% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkte ein gleichmäßigeres Keimen der Testpflanzen, sofern dies nicht durch die Wirkstoffe beeinträchtigt wurde. Die Aufwandmenge für die Vorauflaufanwendung betrug 0,125 oder 0,25 kg/ha a.S. (aktive Substanz).

Zum Zwecke der Nachauflaufbehandlung wurden die Testpflanzen je nach Wuchsform bis zu einer Wuchshöhe von 3 bis 15 cm angezogen und erst dann mit den in Wasser suspendierten oder emulgierten Wirkstoffen behandelt. Die Testpflanzen wurden entweder bereits in den Versuchsgefäßen gesät und aufgezogen, in denen sie behandelt wurden, oder sie wurden als Keimpflanzen getrennt angezogen und einige Tage vor der Behandlung mit den Wirkstoffaufbereitungen in die Versuchsgefäße verpflanzt.

Die Aufwandmenge für die Nachauflaufbehandlung betrug 0,125 oder 0,25 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die Gewächshausversuche wurden an Amaranthus retroflexus, Abutilon theophrasti, Chenopodium album, Matricaria inodora und Veronica subspecies durchgeführt.

Das Ergebnis zeigte, daß mit Verbindung Nr. 1.2 unerwünschte Unkräuter im Nachauflaufverfahren sowie im Vorauflaufverfahren sehr gut bekämpft werden können.

## Patentansprüche

1. Substituierte N-Phenylglutarimide der Formel I 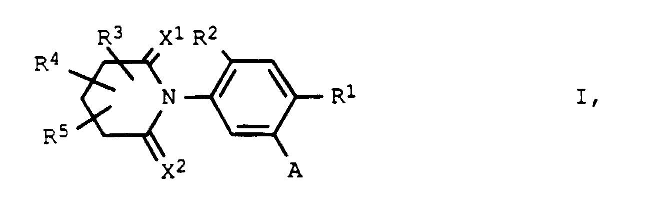 in der die Variablen folgende Bedeutung besitzen:
x¹, x²
Sauerstoff oder Schwefel;
R¹
Halogen, Nitro, Cyano oder Trifluormethyl;
R²
Wasserstoff oder Halogen;
R³, R⁴, R⁵
- unabhängig voneinander Wasserstoff, Halogen, Cyano, C₁-C₆-Alkyl, C₃-C₇-Cycloalkyl, C₂-C₆-Alkenyl, C₂-C₆-Alkinyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Cyanoalkyl, C₁-C₆-Alkoxycarbonyl, Phenyl oder Benzyl, wobei die Phenylgruppe bzw. der Phenylring der Benzylgruppe ggf. substituiert ist durch C₁-C₆-Alkyl, C₁-C₆-Alkoxy, Halogen, Cyano, Nitro, Trifluormethyl,
- oder zwei Substituenten eines Kohlenstoffatoms des Glutarimidrings sind über eine 2- bis 5-gliedrige Kette miteinander verbunden und formen so einen Spiroring, der gewünschtenfalls ein oder zwei Halogenatome tragen kann, wobei der Spiroring neben C-Atomen auch ein oder zwei nicht benachbarte Ringglieder enthalten kann, die ausgewählt sind aus der Gruppe bestehend aus -O-, -S-, -NH-und -N(C₁-C₄-Alkyl)-,
- oder zwei Substituenten zweier benachbarter Kohlenstoffatome des Glutarimidrings sind über eine 1- bis 5-gliedrige Kette miteinander verbunden und bilden einen ankondensierten Ring, der gewünschtenfalls ein oder zwei Halogenatome tragen kann, wobei der ankondensierte Ring neben C-Atomen auch ein oder zwei nicht benachbarte Ringglieder enthalten kann, die ausgewählt sind aus der Gruppe bestehend aus -O-, -S-, -NH- und -N(C₁-C₄-Alkyl)-;
A
CHR⁶-CHR⁷-CO-B oder CR⁶=CR⁸-CO-B, wobei
- R⁶ Wasserstoff, C₁-C₆-Alkyl oder C₁-C₆-Halogenalkyl;
- R⁷ Halogen, C₁-C₆-Halogenalkyl, Hydroxy, C₁-C₆-Alkoxy oder C₁-C₆-Alkylcarbonyloxy, und
- R⁸ Wasserstoff, Halogen, Cyano, C₁-C₆-Alkyl, C₁-C₆-Halogenalkyl, Hydroxy, C₁-C₆-Alkoxy, C₁-C₆-Alkylcarbonyl, C₁-C₆-Alkoxycarbonyl oder C₁-C₆-Alkylcarbonyloxy
bedeuten und wobei
B für eine der folgenden Bedeutungen steht:
(a) Wasserstoff,
(b) C₁-C₆-Alkyl, C₃-C₆-Alkenyl, C₃-C₆-Alkinyl, C₁-C₆-Halogenalkyl, C₃-C₇-Cycloalkyl, C₁-C₆-Alkoxy-C₁-C₆-alkyl, C₁-C₆-Dialkoxy-C₁-C₆-alkyl oder C₁-C₆-Alkylthio-C₁-C₆-alkyl;
(c) OR⁹, SR⁹, wobei R⁹:
Wasserstoff, C₁-C₆-Alkyl, C₃-C₆-Alkenyl, C₃-C₆-Alkinyl, C₃-C₇-Cycloalkyl, Halogen-C₁-C₆-Alkyl, C₁-C₆-Cyanoalkyl, C₃-C₆-Halogenalkenyl, C₁-C₆-Alkoxycarbonyl-C₁-C₆-alkyl, C₁-C₆-Alkoxy-C₁-C₆-alkyl, C₁-C₆-Alkylthio-C₁-C₆-alkyl, C₁-C₆-Alkylimino oder C₁-C₆-Alkyloximino-C₁-C₆-alkyl, Phenyl, Phenyl substituiert durch einen oder mehrere Reste C₁-C₆-Alkyl, C₃-C₆-Alkenyl, Halogen, Cyano, Nitro, C₁-C₆-Alkoxy oder C₁-C₆-Alkoxycarbonyl, Benzyl, Benzyl substituiert durch einen oder mehrere Reste C₁-C₆-Alkyl, C₃-C₆-Alkenyl, Halogen, Cyano, Nitro, C₁-C₆-Alkoxy oder
C₁-C₆-Alkoxycarbonyl bedeutet;
(d) NR¹⁰R¹¹, wobei R¹⁰ und R¹¹ unabhängig voneinander folgende Bedeutung haben:
Wasserstoff, C₁-C₆-Alkyl, C₃-C₆-Alkenyl, C₃-C₆-Alkinyl, C₃-C₇-Cycloalkyl, C₁-C₆-Halogenalkyl, C₁-C₆-Alkylcarbonyl, C₁-C₆-Alkoxycarbonyl, C₁-C₆-Alkoxy-C₁-C₆-alkyl, Phenyl, Phenyl substituiert durch einen bis drei Reste C₁-C₆-Alkyl, C₃-C₆-Alkenyl, Halogen, Cyano, Nitro,
C₁-C₆-Alkoxy oder C₁-C₆-Alkoxycarbonyl, oder R¹⁰ und R¹¹ zusammen mit dem Stickstoffatom, an das sie gebunden sind, einen gesättigten oder ungesättigten 4- bis 7-gliedrigen Heterocyclus mit einem oder zwei weiteren gleichen oder verschiedenen Heteroatomen ausgewählt aus der Gruppe Stickstoff, Sauerstoff oder Schwefel;
sowie die landwirtschaftlich brauchbaren Salze der substituierten N-Phenylglutarimide I.
2. Substituierte N-Phenylglutarimide der Formel I gemäß Anspruch 1, in der R⁴ C₁-C₆-Alkyl oder C₁-C₆-Halogenalkyl und R³ und R⁵ Wasserstoff oder C₁-C₄-Alkyl bedeuten.
3. Substituierte N-Phenylglutarimide der Formel I' 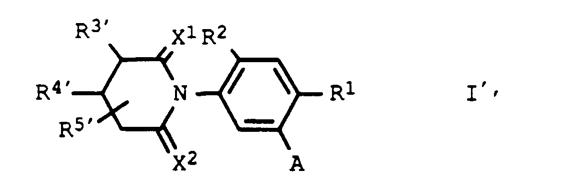 wobei R⁴' C₁-C₆-Halogenalkyl, R³'und R⁵' unabhängig voneinander Wasserstoff, C₁-C₆-Alkyl oder C₁-C₆-Halogenalkyl bedeuten und A, X¹, X² wie in Anspruch 1 definiert sind.
4. Substituierte N-Phenylglutarimide der Formel I' gemäß Anspruch 3, in der R⁴ Trichlormethyl, Difluormethyl, Trifluormethyl, Pentafluorethyl oder Difluorchlormethyl bedeutet.
5. Substituierte N-Phenylglutarimide der Formel I gemäß Anspruch 1, wobei R¹ Halogen bedeutet.
6. Substituierte N-Phenylglutarimide der Formel I gemäß Anspruch 1, wobei R² Wasserstoff, Fluor oder Chlor bedeutet.
7. Substituierte N-Phenylglutarimide der Formel I gemäß Anspruch 1, wobei A CHR⁶-CHR⁷-CO-B bedeutet.
8. Substituierte N-Phenylglutarimide der Formel I gemäß Anspruch 1, wobei A CR⁶=CR⁸-CO-B bedeutet.
9. N-Phenylglutarsäureamide der allgemeinen Formel II 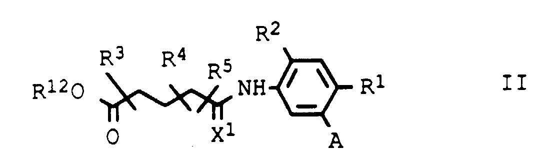 in der X¹, R¹, R², R³, R⁴, R⁵ und A die in Anspruch 1 genannte Bedeutung haben und R¹² Wasserstoff, C₁-C₆-Alkyl oder Benzyl steht, sowie die landwirtschaftlich brauchbaren Salze der N-Phenylglutarsäureamide II.
10. Herbizides Mittel oder Mittel zur Desikkation und/oder Defoliation von Pflanzen, enthaltend einen inerten flüssigen oder festen Trägerstoff und eine herbizid bzw. desikkant und/ oder defoliant wirksame Menge mindestens eines substituiertes N-Phenylglutarimids der Formel I oder eines landwirtschaftlich brauchbaren Salzes von I gemäß Anspruch 1.
11. Verfahren zur Bekämpfung von unerwünschtem Pflanzenwuchs, dadurch gekennzeichnet, daß man eine herbizid wirksame Menge mindestens eines substituierten N-Phenylglutarimids der Formel I oder ein landwirtschaftlich brauchbaren Salzes von I gemäß Anspruch 1, auf Pflanzen, deren Lebensraum oder auf Saatgut einwirken läßt.
12. Verfahren zur Desikkation und/oder Defoliation von Pflanzen, dadurch gekennzeichnet, daß man eine desikkant und/oder defoliant wirksame Menge mindestens eines substituierten N-Phenylglutarimids der Formel I oder eines landwirtschaftlich brauchbaren Salzes von I gemäß Anspruch 1 auf Pflanzen einwirken läßt.
13. Herbizides Mittel oder Mittel zur Desikkation und/oder Defoliation von Pflanzen, enthaltend einen inerten flüssigen oder festen Trägerstoff und eine herbizid bzw. desikkant und/ oder defoliant wirksame Menge mindestens eines N-Phenylglutarsäureamids der Formel II oder eines landwirtschaftlich brauchbaren Salzes von II gemäß Anspruch 9.
14. Verfahren zur Bekämpfung von unerwünschtem Pflanzenwuchs, dadurch gekennzeichnet, daß man eine herbizid wirksame Menge mindestens eines N-Phenylglutarsäureamids der Formel II oder eines landwirtschaftlich brauchbaren Salzes von II gemäß Anspruch 9 auf Pflanzen, deren Lebensraum oder auf Saatgut einwirken läßt.
15. Verfahren zur Desikkation und/oder Defoliation von Pflanzen, dadurch gekennzeichnet, daß man eine defoliant und/oder desiccant wirksame Menge mindestens eines N-Phenylglutarsäureamids der Formel II oder eines landwirtschaftlich brauchbaren Salzes von II gemäß Anspruch 9 auf Pflanzen einwirken läßt.
16. Verfahren zur Herstellung von substituierten N-Phenylglutarimiden der Formel I gemäß Anspruch 1,
a) mit X¹= Sauerstoff oder Schwefel und X² = Sauerstoff, dadurch gekennzeichnet, daß man ein N-Phenylglutarsäureamid der Formel II gemäß Anspruch 9, 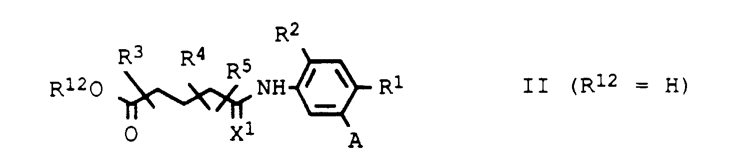 in der R¹² für Wasserstoff steht, in Gegenwart eines Dehydratisierungsmittels cyclisiert;
b) mit X¹ = Sauerstoff oder Schwefel und X² = Sauerstoff, dadurch gekennzeichnet, daß man ein N-Phenylglutarsäureamids der Formel II gemäß Anspruch 9, 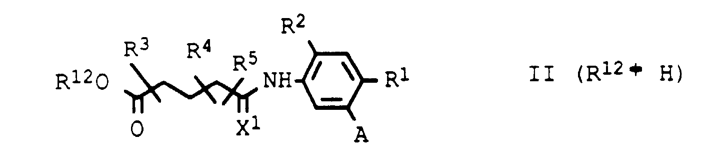 cyclisiert;
c) mit X¹ = Schwefel und X² = Sauerstoff oder Schwefel, dadurch gekennzeichnet, daß man ein N-Phenylglutarimid der Formel I, wobei X¹ Sauerstoff bedeutet, 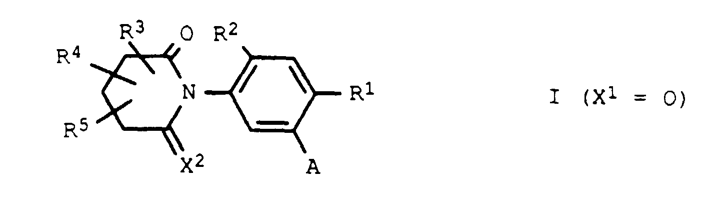 mit einem geeigneten Schwefelungsreagenz umsetzt.
17. Verfahren zur Herstellung von N-Phenylglutaramiden der Formel II gemäß Anspruch 9
d) mit R¹² = H und X² = Sauerstoff, dadurch gekennzeichnet, daß man ein Anilin der Formel III 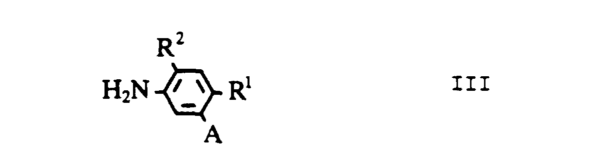 mit einem Glutarsäureanhydrid der Formel IV 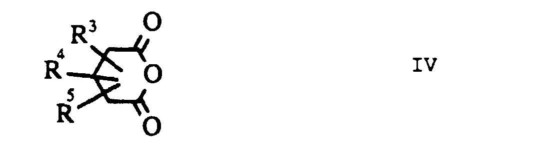 umsetzt;
e) mit R¹² + H und X² = Sauerstoff, dadurch gekennzeichnet, daß man ein Anilin der Formel III mit einem Glutarsäuremonoester der Formel V 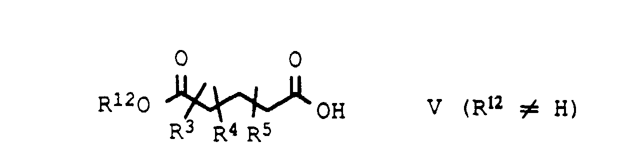 in Gegenwart einer Base umsetzt;
f) mit X¹ = Schwefel, dadurch gekennzeichnet, daß man eine Verbindung der Formel II, wobei X¹ Sauerstoff bedeutet, 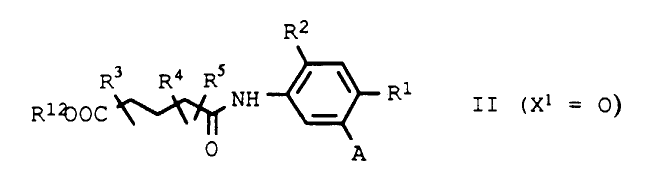 mit einem geeigneten Schwefelungsreagenz umsetzt.

## Claims

1. A substituted N-phenylglutarimide of the formula I 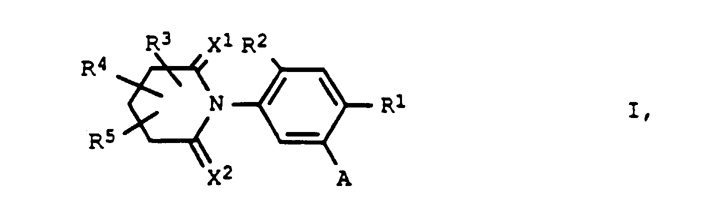 where the variables have the following meanings:
x¹, x²
are oxygen or sulfur;
R¹
is halogen, nitro, cyano or trifluoromethyl;
R²
is hydrogen or halogen;
R³, R⁴, R⁵
- independently of one another are hydrogen, halogen, cyano, C₁-C₆-alkyl, C₃-C₇-cycloalkyl, C₂-C₆-alkenyl, C₂-C₆-alkynyl, C₁-C₆-haloalkyl, C₁-C₆-alkoxy, C₁-C₆-haloalkoxy, C₁-C₆-alkylthio, C₁-C₆-haloalkylthio, C₁-C₆-cyanoalkyl, C₁-C₆-alkoxycarbonyl, phenyl or benzyl, where the phenyl group or the phenyl ring of the benzyl group may be substituted by C₁-C₆-alkyl, C₁-C₆-alkoxy, halogen, cyano, nitro or trifluoromethyl,
- or two substituents of a carbon atom of the glutarimide ring are bonded to one another via a 2-to 5-membered chain and thus form a spiro ring, which may, if desired, carry one or two halogen atoms, it being possible for the spiro ring to contain, beside C atoms, also one or two nonadjacent ring members which are selected from the group consisting of -O-, -S-, -NH- and -N(C₁-C₄-alkyl)-,
- or two substituents of two adjacent carbon atoms of the glutarimide ring are bonded to one another via a 1- to 5-membered chain and thus form a fused ring, which may, if desired, carry one or two halogen atoms, it being possible for the fused ring to contain, beside C atoms, also one or two nonadjacent ring members which are selected from the group consisting of -O-, -S-, -NH- and -N(C₁-C₄-alkyl)-;
A
is CHR⁶-CHR⁷-CO-B or CR⁶=CR⁸-CO-B, where
- R⁶ is hydrogen, C₁-C₆-alkyl or C₁-C₆-haloalkyl;
- R⁷ is halogen, C₁-C₆-haloalkyl, hydroxyl, C₁-C₆-alkoxy or C₁-C₆-alkylcarbonyloxy, and
- R⁸ is hydrogen, halogen, cyano, C₁-C₆-alkyl, C₁-C₆-haloalkyl, hydroxyl, C₁-C₆-alkoxy, C₁-C₆-alkylcarbonyl, C₁-C₆-alkoxycarbonyl or C₁-C₆-alkylcarbonyloxy
and where
B has one of the following meanings:
(a) hydrogen,
(b) C₁-C₆-alkyl, C₃-C₆-alkenyl, C₃-C₆-alkynyl, C₁-C₆-haloalkyl, C₃-C₇-cycloalkyl, C₁-C₆-alkoxy-C₁-C₆-alkyl, C₁-C₆-dialkoxy-C₁-C₆-alkyl or C₁-C₆-alkylthio-C₁-C₆-alkyl;
(c) OR⁹, SR⁹, where R⁹ is:
hydrogen, C₁-C₆-alkyl, C₃-C₆-alkenyl, C₃-C₆-alkynyl, C₃-C₇-cycloalkyl, halo-C₁-C₆-alkyl, C₁-C₆-cyanoalkyl, C₃-C₆-haloalkenyl, C₁-C₆-alkoxycarbonyl-C₁-C₆-alkyl, C₁-C₆-alkoxy-C₁-C₆-alkyl, C₁-C₆-alkylthio-C₁-C₆-alkyl, C₁-C₆-alkylimino or C₁-C₆-alkyloximino-C₁-C₆-alkyl, phenyl, phenyl substituted by one or more C₁-C₆-alkyl, C₃-C₆-alkenyl, halogen, cyano, nitro, C₁-C₆-alkoxy or C₁-C₆-alkoxycarbonyl radicals, benzyl, benzyl substituted by one or more C₁-C₆-alkyl, C₃-C₆-alkenyl, halogen, cyano, nitro, C₁-C₆-alkoxy or C₁-C₆-alkoxycarbonyl radicals;
(d) NR¹⁰R¹¹, where R¹⁰ and R¹¹ independently of one another have the following meanings:
hydrogen, C₁-C₆-alkyl, C₃-C₆-alkenyl, C₃-C₆-alkynyl, C₃-C₇-cycloalkyl, C₁-C₆-haloalkyl, C₁-C₆-alkylcarbonyl, C₁-C₆-alkoxycarbonyl, C₁-C₆-alkoxy-C₁-C₆-alkyl, phenyl, phenyl substituted by one to three C₁-C₆-alkyl, C₃-C₆-alkenyl, halogen, cyano, nitro, C₁-C₆-alkoxy or C₁-C₆-alkoxycarbonyl radicals, or Rand R¹¹-, together with the nitrogen atom to which they are bonded, are a saturated or unsaturated 4-to 7- membered heterocycle having one or two further identical or different heteroatoms selected from the group consisting of nitrogen, oxygen and sulfur;
or an agriculturally utilizable salt of the substituted N-phenylglutarimide I.

2. A substituted N-phenylglutarimide of the formula I as claimed in claim 1, where R⁴ is C₁-C₆-alkyl or C₁-C₆-haloalkyl and R³ and R⁵ are hydrogen or C₁-C₄-alkyl.

3. A substituted N-phenylglutarimide of the formula I, 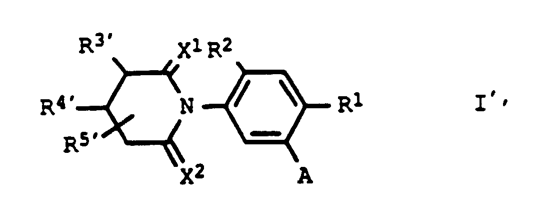 where R⁴' is C₁-C₆-haloalkyl, R³' and R⁵' independently of one another are hydrogen, C₁-C₆-alkyl or C₁-C₆-haloalkyl and A, X¹ and X² are as defined in claim 1.

4. A substituted N-phenylglutarimide of the formula I' as claimed in claim 3, where R⁴' is trichloromethyl, difluoromethyl, trifluoromethyl, pentafluoroethyl or difluorochloromethyl.

5. A substituted N-phenylglutarimide of the formula I as claimed in claim 1, where R¹ is halogen.

6. A substituted N-phenylglutarimide of the formula I as claimed in claim 1, where R² is hydrogen, fluorine or chlorine.

7. A substituted N-phenylglutarimide of the formula I as claimed in claim 1, where A is CHR⁶-CHR⁷-CO-B.

8. A substituted N-phenylglutarimide of the formula I as claimed in claim 1, where A is CR⁶=CR⁸-CO-B.

9. An N-phenylglutaramide of the general formula II 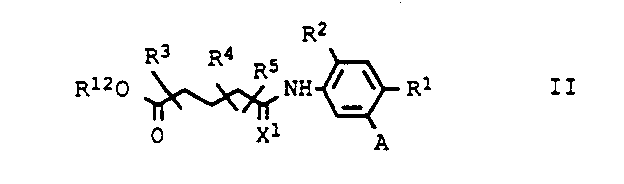 where x¹, R¹, R², R³, R⁴, R⁵ and A have the meanings mentioned in claim 1 and R¹² is hydrogen, C₁-C₆-alkyl or benzyl, or an agriculturally utilizable salt of the N-phenylglutaramide II.

10. A herbicidal composition or composition for the desiccation and/or defoliation of plants, containing an inert, liquid or solid carrier and an amount of at least one substituted N-phenylglutarimide of the formula I or of an agriculturally utilizable salt of I as claimed in claim 1 which is herbicidally active or has desiccant and/or defoliant activity.

11. A method for combating undesired plant growth, which comprises allowing an amount of at least one substituted N-phenylglutarimide of the formula I or of an agriculturally utilizable salt of I as claimed in claim 1 which is herbicidally active to act on plants, their environment or on seeds.

12. A method for the desiccation and/or defoliation of plants, which comprises allowing an amount of at least one substituted N-phenylglutarimide of the formula I or of an agriculturally utilizable salt of I as claimed in claim 1 which has desiccant and/or defoliant activity to act on plants.

13. A herbicidal composition or composition for the desiccation and/or defoliation of plants, containing an inert, liquid or solid carrier and an amount of at least one N-phenylglutaramide of the formula II or of an agriculturally utilizable salt of II as claimed in claim 9 which is herbicidally active or has desiccant and/or defoliant activity.

14. A method for combating undesired plant growth, which comprises allowing an amount of at least one N-phenylglutaramide of the formula II or of an agriculturally utilizable salt of II as claimed in claim 9 which is herbicidally active to act on plants, their environment or on seeds.

15. A method for the desiccation and/or defoliation of plants, which comprises allowing an amount of at least one N-phenylglutaramide of the formula II or of an agriculturally utilizable salt of II as claimed in claim 9 which has defoliant and/or desiccant activity to act on plants.

16. A process for preparing substituted N-phenylglutarimides of the formula I as claimed in claim 1
a) where X¹ = oxygen or sulfur and X² = oxygen, which comprises cyclizing an N-phenylglutaramide of the formula II as claimed in claim 9, 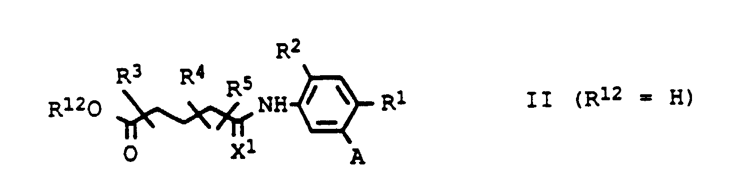 where R¹² is hydrogen, in the presence of a dehydrating agent;
b) where X¹ = oxygen or sulfur and X² = oxygen, which comprises cyclizing an N-phenylglutaramide of the formula II as claimed in claim 9, 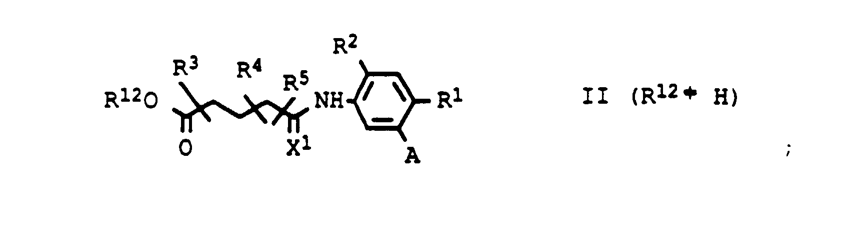
c) where X¹ = sulfur and X² = oxygen or sulfur, which comprises reacting an N-phenylglutarimide of the formula I, where X¹ is oxygen, 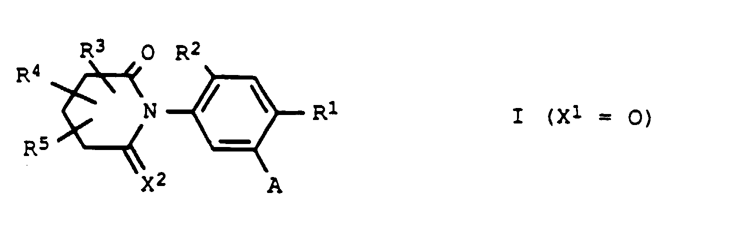 with a suitable sulfurizing reagent.

17. A process for preparing N-phenylglutaramides of the formula II as claimed in claim 9
d) where R¹² = H and X² = oxygen, which comprises reacting an aniline of the formula III 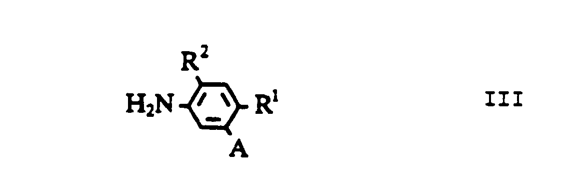 with a glutaric anhydride of the formula IV 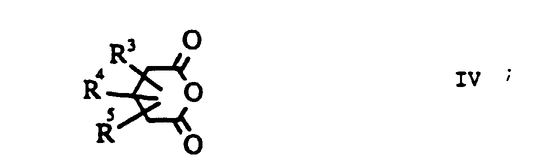
e) where R¹² ≠ H and X² = oxygen, which comprises reacting an aniline of the formula III with a glutaric acid monoester of the formula V 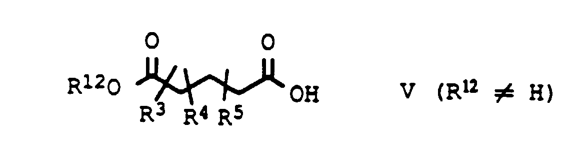 in the presence of a base;
f) where X¹ = sulfur, which comprises reacting a compound of the formula II, where X¹ is oxygen, 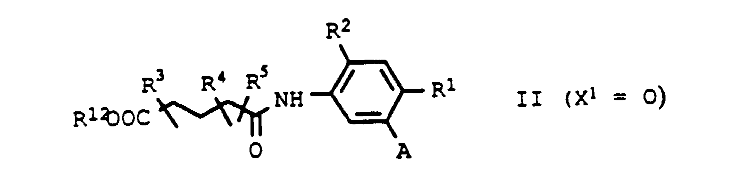 with a suitable sulfurizing reagent

## Revendications

1. N-phénylglutarimides substitués de formule I 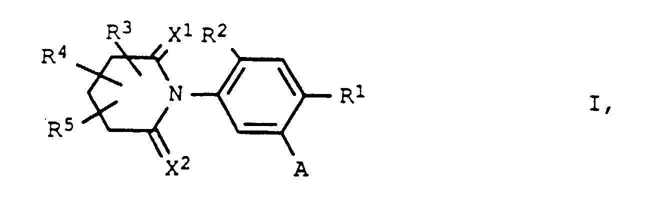 dans laquelle les symboles ont les significations suivantes :
X¹, X² :
l'oxygène ou le soufre ;
R¹ :
un halogène, un groupe nitro, cyano ou trifluorométhyle ;
R² :
l'hydrogène ou un halogène ;
R³, R⁴, R⁵ :
- indépendamment les uns des autres : l'hydrogène, un halogène, un groupe cyano, alkyle en C1-C6, cycloalkyle en C3-C7, alcényle en C2-C6, alcynyle en C2-C6, halogénoalkyle en C1-C6, alcoxy en C1-C6, halogénoalcoxy en C1-C6, alkylthio en C1-C6, halogénoalkylthio en C1-C6, cyanoalkyle en C1-C6, (alcoxy en C1-C6)carbonyle, phényle ou benzyle, le groupe phényle et le cycle phényle du groupe benzyle pouvant être substitués le cas échéant par des groupes alkyle en C1-C6, alcoxy en C1-C6, des halogènes, des groupes cyano, nitro, trifluorométhyle,
- ou bien deux substituants d'un atome de carbone du cycle glutarimide sont reliés entre eux par une chaîne de deux à cinq chaînons et forment ainsi un spirocycle qui peut porter si on le désire un ou deux atomes d'halogènes, le spirocycle pouvant contenir, avec les atomes de carbone, un ou deux chaînons cycliques non voisins choisis parmi -O-, -S-, -NH- et -N(alkyle en C1-C4)-,
- ou bien deux substituants de deux atomes de carbone voisins du cycle glutarimide sont reliés entre eux par une chaîne de un à cinq chaînons et forment un cycle condensé qui, si on le désire, peut porter un ou deux atomes d'halogènes, le cycle condensé pouvant contenir, en plus des atomes de carbone, un ou deux chaînons cycliques non voisins choisis parmi -O-, -S-, -NH- et -N(alkyle en C1-C4)- ;
A:
CHR⁶-CHR⁷-CO-B ou CR⁶=CR⁸-CO-B dans lesquels
- R⁶ représente l'hydrogène, un groupe alkyle en C1-C6 ou halogénoalkyle en C1-C6;
- R⁷ représente un halogène, un groupe halogénoalkyle en C1-C6, hydroxy, alcoxy en C1-C6 ou (alkyle en C1-C6)carbonyloxy et
- R⁸ représente l'hydrogène, un halogène, un groupe cyano, alkyle en C1-C6, halogénoalkyle en C1-C6, hydroxy, alcoxy en C1-C6, (alkyle en C1-C6)carbonyle, (alcoxy en C1-C6)carbonyle ou (alkyle en C1-C6)carbonyloxy et
B a l'une des significations suivantes :
(a) l'hydrogène,
(b) un groupe alkyle en C1-C6, alcényle en C3-C6, alcynyle en C3-C6, halogénoalkyle en C1-C6, cycloalkyle en C3-C7, (alcoxy en C1-C6)alkyle en C1-C6, di-(alcoxy en C1-C6)alkyle en C1-C6 ou (alkylthio en C1-C6)alkyle en C1-C6;
(c) OR⁹, SR⁹ dans lesquels R⁹ représente :
l'hydrogène, un groupe alkyle en C1-C6, alcényle en C3-C6, alcynyle en C3-C6, cycloalkyle en C3-C7, halogénoalkyle en C1-C6, cyanoalkyle en C1-C6, halogénoalcényle en C3-C6, (alcoxy en C1-C6)carbonyl-alkyle en C1-C6, (alcoxy en C1-C6)alkyle en C1-C6, (alkylthio en C1-C6)alkyle en C1-C6, alkylimino en C1-C6 ou (alkyle en C1-C6)oximino-alkyle en C1-C6, phényle, phényle portant un ou plusieurs substituants alkyle en C1-C6, alcényle en C3-C6, halogéno, cyano, nitro, alcoxy en C1-C6 ou (alcoxy en C1-C6)carbonyle, benzyle, benzyle portant un ou plusieurs substituants alkyle en C1-C6, alcényle en C3-C6, halogéno, cyano, nitro, alcoxy en C1-C6 ou (alcoxy en C1-C6)carbonyle ;
(d) NR¹⁰R¹¹ dans lequel R¹⁰ et R¹¹ ont chacun, indépendamment l'un de l'autre, l'une des significations suivantes :
l'hydrogène, un groupe alkyle en C1-C6, alcényle en C3-C6, alcynyle en C3-C6, cycloalkyle en C3-C7, halogénoalkyle en C1-C6, (alkyle en C1-C6)carbonyle, (alcoxy en C1-C6)carbonyle, (alcoxy en C1-C6)alkyle en C1-C6, phényle, phényle portant un à trois substituants alkyle en C1-C6, alcényle en C3-C6, halogéno, cyano, nitro, alcoxy en C1-C6 ou (alcoxy en C1-C6)carbonyle, ou bien R¹⁰ et R¹¹ forment ensemble et avec l'atome d'azote auquel ils sont reliés un hétérocycle saturé ou insaturé de quatre à sept chaînons contenant un ou deux autres hétéroatomes identiques ou différents choisis parmi l'azote, l'oxygène et le soufre ;
ainsi que les sels aptes aux applications agricoles des N-phénylglutarimides substitués I.

2. N-phénylglutarimides substitués de formule I selon la revendication 1 dans laquelle R⁴ représente un groupe alkyle en C1-C6 ou halogénoalkyle en C1-C6 et R³ et R⁵ représentent l'hydrogène ou des groupes alkyle en C1-C4.

3. N-phénylglutarimides substitués de formule I' 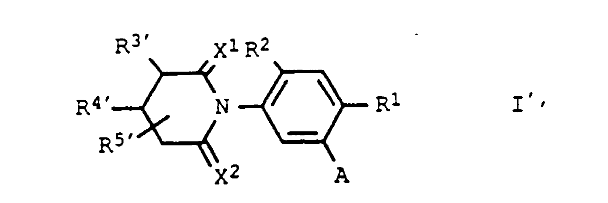 dans laquelle R⁴' représente un groupe halogénoalkyle en C1-C6, R³, et R⁵' représentent chacun, indépendamment l'un de l'autre, l'hydrogène, un groupe alkyle en C1-C6 ou halogénoalkyle en C1-C6 et A, X¹ et X² ont les significations indiquées dans la revendication 1.

4. N-phénylglutarimides substitués de formule I' de la revendication 3, dans laquelle R⁴ représente un groupe trichlorométhyle, difluorométhyle, trifluorométhyle, pentafluoréthyle ou difluorochlorométhyle.

5. N-phénylglutarimides substitués de formule I de la revendication 1, dans laquelle R¹ représente un halogène.

6. N-phénylglutarimides substitués de formule I de la revendication 1, dans laquelle R² représente l'hydrogène, le fluor ou le chlore.

7. N-phénylglutarimides substitués de formule I de la revendication 1, dans laquelle A représente CHR⁶-CHR⁷-CO-B.

8. N-phénylglutarimides substitués de formule I de la revendication 1, dans laquelle A représente CR⁶=CR⁸-CO-B.

9. N-phénylglutarimides de formule générale II 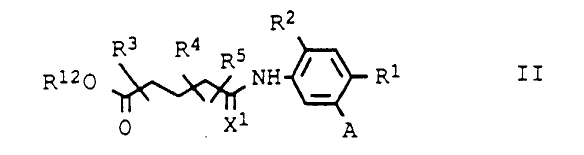 dans laquelle X¹, R¹, R², R³, R⁴, R⁵ et A ont les significations indiquées dans la revendication 1 et R¹² représente l'hydrogène, un groupe alkyle en C1-C6 ou benzyle, et les sels aptes à des applications agricoles des N-phénylglutarimides II.

10. Produit herbicide ou produit pour la dessication et/ou la défoliation des végétaux, contenant un véhicule solide ou liquide inerte et une quantité herbicide ou dessiccative et/ou défoliante efficace d'au moins un N-phénylglutarimide substitué de formule I ou d'un sel, apte aux applications agricoles, de I selon la revendication 1.

11. Procédé pour combattre les croissances de végétaux indésirables, caractérisé par le fait que l'on fait agir une quantité herbicide efficace d'au moins un N-phénylglutarimide substitué de formule I ou d'un sel apte aux applications agricoles de I selon la revendication 1 sur les végétaux, leur habitat ou les semences.

12. Procédé pour la dessication et/ou la défoliation de végétaux, caractérisé par le fait que l'on fait agir sur les végétaux une quantité dessiccative et/ou défoliante efficace d'au moins un N-phénylglutarimide substitué de formule I ou d'un sel apte aux applications agricoles de I selon la revendication 1.

13. Produit herbicide ou produit pour la dessication et/ou la défoliation des végétaux, contenant un véhicule liquide ou solide inerte et une quantité herbicide ou dessiccative et/ou défoliante efficace d'au moins un N-phénylglutarimide de formule II ou d'un sel apte aux applications agricoles de II selon la revendication 9.

14. Procédé pour combattre les croissances de végétaux indésirables, caractérisé par le fait que l'on fait agir sur les végétaux, leur habitat ou les semences une quantité herbicide efficace d'au moins un N-phénylglutarimide de formule II ou d'un sel apte aux applications agricoles de II selon la revendication 9.

15. Procédé pour la dessiccation et/ou la défoliation des végétaux, caractérisé par le fait que l'on fait agir sur les végétaux une quantité défoliante et/ou dessiccative efficace d'au moins un N-phénylglutarimide de formule II ou d'un sel apte aux applications agricoles de II selon la revendication 9.

16. Procédé de préparation des N-phénylglutarimides substitués de formule I de la revendication 1,
a) dans laquelle X¹ = oxygène ou soufre et X² = oxygène,
caractérisé par le fait que l'on cyclise un N-phénylglutarimide de formule II de la revendication 9, 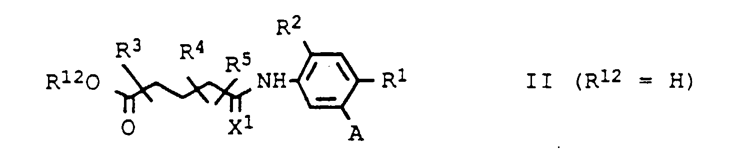 dans laquelle R¹² représente l'hydrogène, en présence d'un agent déshydratant ;
b) dans laquelle X¹ = oxygène ou soufre et X² = oxygène,
caractérisé par le fait que l'on cyclise un N-phénylglutarimide de formule II de la revendication 9 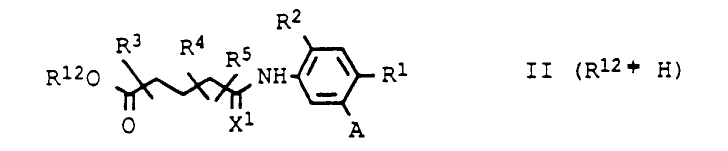
c) dans laquelle X¹ = soufre et X² = oxygène ou soufre,
caractérisé par le fait que l'on fait réagir un N-phénylglutarimide de formule I dans laquelle X¹ représente l'oxygène, 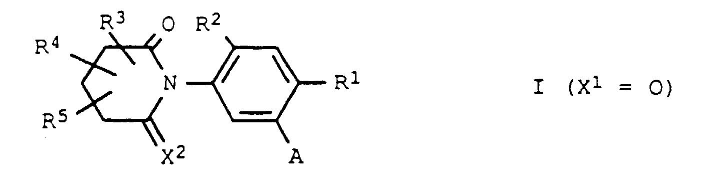 avec un réactif sulfurant approprié.

17. Procédé de préparation des N-phénylglutarimides de formule II de la revendication 9
d) dans laquelle R¹² = H et X² = oxygène,
caractérisé par le fait que l'on fait réagir une aniline de formule III 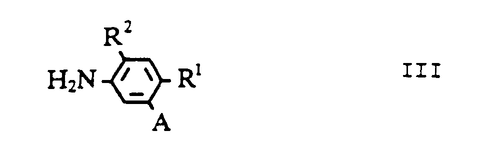 avec un anhydride glutarique de formule IV 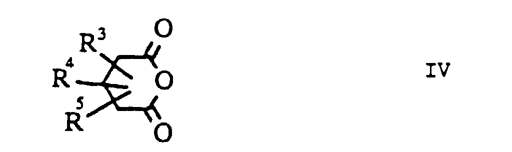
e) dans laquelle R¹² ≠ H et X² = oxygène, caractérisé par le fait que l'on fait réagir une aniline de formule III avec un monoester glutarique de formule V 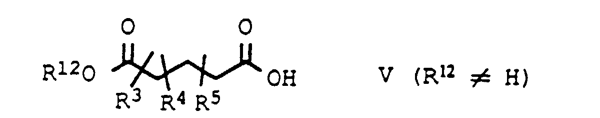 en présence d'une base ;
f) dans laquelle X¹ = soufre, caractérisé par le fait que l'on fait réagir un composé de formule II dans laquelle X¹ représente l'oxygène 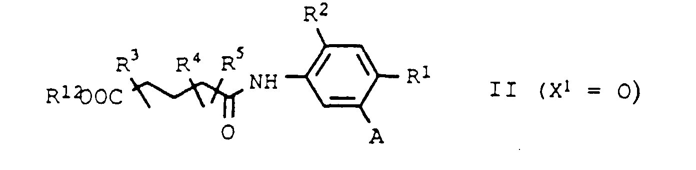 avec un réactif sulfurant approprié.
